# EUROPEAN PATENT SPECIFICATION

(11) **EP 3 906 846 B1**
(45) Date of publication and mention of the grant of the patent: **20.03.2024**
(21) Application number: 21155664.2
(22) Date of filing: 08.02.2021
(51) Int. Cl.: A61B 5/0205, A61B 5/346, A61B 5/08, A61B 5/00, A61B 5/11

(54) **METHOD AND DEVICE FOR DETECTING RESPIRATION ANOMALY FROM LOW FREQUENCY COMPONENT OF ELECTRICAL CARDIAC ACTIVITY SIGNALS**
VERFAHREN UND VORRICHTUNG ZUR DETEKTION VON ATMUNGSANOMALIEN AUS EINER NIEDERFREQUENTEN KOMPONENTE VON ELEKTRISCHEN HERZAKTIVITÄTSSIGNALEN
PROCÉDÉ ET DISPOSITIF DE DÉTECTION D'ANOMALIE DE RESPIRATION À PARTIR D'UN COMPOSANT À BASSE FRÉQUENCE DE SIGNAUX D'ACTIVITÉ CARDIAQUE

(30) Priority: 08.05.2020 US 202016869733
(43) Date of publication of application: 10.11.2021
(73) Proprietor: Pacesetter, Inc., Sylmar, CA 91342 (US)
(72) Inventor: GILL, Jong, Valencia, CA 91355 (US); ANDERSEN, Dean P., San Jose, CA 95120 (US)
(74) Representative: Barker Brettell Sweden AB

(56) References cited:
- US-A1- 2012 101 393
- US-A1- 2019 133 457
- US-B1- 7 628 757
- US-B1- 8 262 578

## Description

### BACKGROUND

Embodiments of the present disclosure generally relate to methods and devices for collecting and analyzing respiration components within electrical cardiac activity signals, and more particularly to methods and devices for detecting respiration anomalies based thereon.

Individuals experience various breathing anomalies, such as sleep apnea and hypopnea. Sleep apnea is a rather common disorder with diffuse symptoms. Usually during daytime the patient experiences fatigue, concentration problems and problems of staying awake. At night, the patient's sleep is disrupted by episodes of apnea, usually caused by the epiglottis falling back and obstructing the airways. The apnea causes the person to awake thus disrupting the normal sleep pattern. Sleep apnea syndrome (SAS), which is characterized by repeated episodes of reduced (hypopnea) or absent (apnea) airflow, is a common disorder affecting roughly 50% among middle-aged men.

Monitoring patient respiration can be desirable in children as well as in adults. Sudden infant death syndrome (SIDS) is, for example, one of the most common causes of death among infants under the age of one year. During the night infants normally experience apnea. A healthy infant will awake so as to resume breathing if the apnea lasts too long. If the infant is unable to awake itself, however, accidental suffocation and sudden death can occur. The reason for the inability of some infants to awaken themselves and the etiology of SIDS is to a large extent unknown but some correlation to rotavirus infection has been found. The clinical manifestation consists, as mentioned, in interruptions of the breathing of the infant during sleep and as a consequence death of the infant.

Various conventional systems have been proposed for sensing respiration activity. For example, systems for sensing respiration activity have been proposed based on the collection of heart sounds, accelerometer signals, and photoplethysmography (PPG) signals. U.S. Patent 6,064,910 (commonly assigned with the present application) describes a device for determining the respiration rate and/or respiration depth of a patient that includes a sensor for sensing heart sounds and an analyzer for analyzing the variation of the amplitude of the sensed heart sounds to determine the respiration rate and/or respiration depth from this amplitude variation. In another approach, U.S. Patent 7,678,061 describes a system and method for characterizing patient respiration based on transthoracic impedance that is derived from a transthoracic impedance sensor. In another approach, U.S. Patent 9,022,030 monitors respiratory disorders based on photoplethysmography (PPG) signals that are representative of peripheral blood volume.

However, these conventional systems experience certain limitations. For example, activity signals collected by accelerometers exhibit large artifacts due to movement and changes in patient orientation, where such artifacts render it difficult to accurately extract only the signal components related to respiration activity, due in part to the fact that movement also induces a low-frequency signal component into the activity signal. Also, it is difficult to derive tidal volume within an individual breath from accelerometer signals. As another example, PPG signals collected by PPG sensors utilize separate configurations and sensing channels and require a higher power demand.

A need remains for methods and devices that are able to monitor and detect respiratory anomalies in a reliable manner and through the inclusion of a relatively simple low-power system.

US 7,628,757 discloses techniques for detecting pulmonary edema based on a comparison of impedance-based respiratory patterns and impedance-based cardiac patterns, i.e. patterns derived from thoracic impedance signals. In one example, a numerical ratio is calculated between average peak-to-peak amplitudes of the respiratory patterns and average peak-to-peak amplitudes of the cardiac patterns. Pulmonary edema is detected if the amplitude ratio falls below a pulmonary edema detection threshold. Techniques are also provided for controlling operation of an impedance-based reduced respiration detector, i.e. a detector which seeks to detect apnea, hypopnea, or the like, based on analysis of respiratory patterns derived from a thoracic impedance signal. If the numerical ratio falls below a minimum reliability threshold, then the reduced respiration detector is deactivated because episodes of reduced respiration cannot then reliably be derived from an analysis of respiration patterns obtained from the thoracic impedance signals.

US 2012/0101393 discloses a method and apparatus for monitoring respiration in a patient sense a cardiac electrical signal and detect signal peaks from the cardiac electrical signal. A peak amplitude waveform is generated from the signal peaks. a first derivative of the peak amplitude waveform is computed. Inspiration pulses are derived from the first derivative signal, and a respiration metric can be computed using the inspiration pulses derived from the cardiac electrical signal.

### SUMMARY

The invention is as specified in the appended claims.

In accordance with new and unique aspects herein, methods and devices are described that provide a relatively limited modification of an existing implantable medical device that enables the existing implantable medical device to monitor respiration components, that are low frequency components within a cardiac activity signal, and identify respiration anomalies in a reliable manner. The modifications described herein place relatively low power demand upon the existing medical device. By enabling the existing implantable medical device to collect and identify new information, namely respiration anomalies, improvements herein collect and provide clinicians with valuable information for proper clinical care. However, embodiments herein are not limited to implementations that modify an existing medical device. Instead, embodiments may be implemented in connection with entirely new devices.

In accordance with new and unique aspects herein, it is been recognized that an additional helpful signal component can be derived from the intracardiac electrogram (IEGM) signals that are already captured by implantable medical devices and/or derived from electrocardiogram (ECG) signals that are captured by wearable devices. For example, the implantable medical device may represent a pacemaker. The prevalence of SAS in patients that have a pacemaker is relatively high (up to 50%). Implantable medical devices are already able to capture IEGM signals in a low-power manner using simple sensor configurations, thereby avoiding the need for any additional sensors or complex sensing circuitry.

In accordance with new and unique aspects herein, it is been recognized that the additional signal components that can be derived from the IEGM/ECG signals also exhibit very low susceptibility to artifacts that may be present in other types of sensors systems that detect respiration activity.

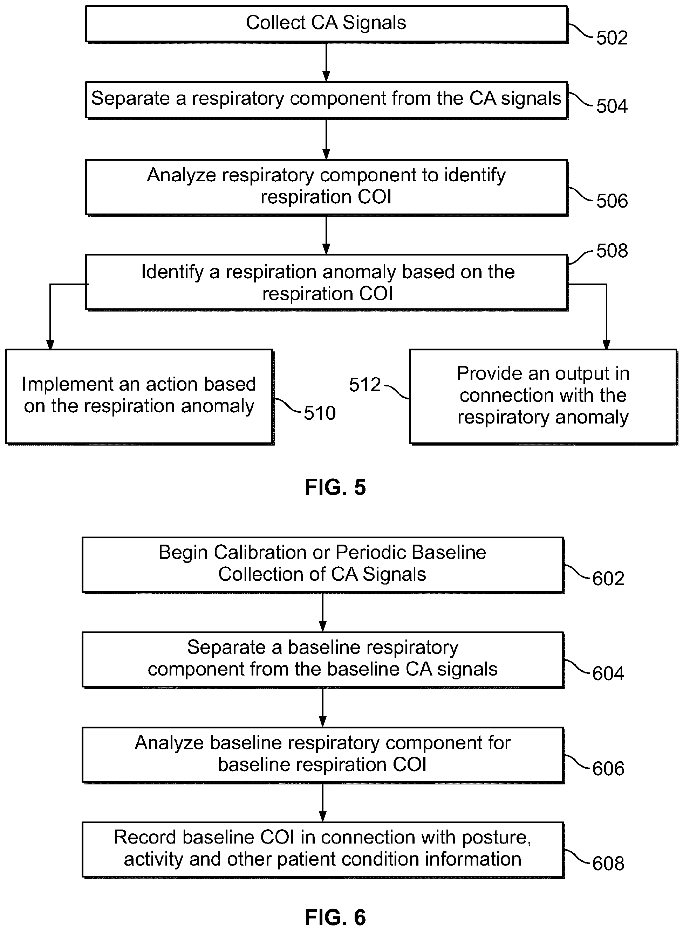

In accordance with new and unique aspects herein, it has been found that posture can impact IEGM/ECG signal amplitude and therefore it can be important to manage when to perform the operations described herein relative to posture. The CA signals (e.g., IEGM/ECG) may be collected when patients are inactive or asleep to minimize the effect that activity or posture has on the CA signals. Embodiments herein utilize 3D accelerometer signals to monitor activity level as well as posture measurements. One or more processors of the IMD may determine that the activity level has dropped below a lower threshold and remained below the lower threshold for a select period of time, thereby indicating that the patient is asleep. Additionally or alternatively, the one or more processors may determine that the patient posture is supine and has remained supine for a select period of time, as another indicator that the patient is asleep. Based on one or both of the activity level and/or posture, the one or more processors may determine that the time is appropriate for the process of Figure 5 to be implemented to filter respiration components from the IEGM/ECG signals and utilize the respiration components to identify respiratory anomalies.

While embodiments herein generally discuss respiration anomalies in connection with small tidal volume or long intervals between breaths, it is recognized that the present application is not limited thereto. Additionally or alternatively, the respiration anomaly may represent breathing too fast, such as when experiencing a shortness of breath or when experiencing difficulties breathing. Embodiments herein may search for respiration pattern characteristics indicative of shortness of breath or breathing difficulties alone or in combination with other physiologic data collected by the medical device and/or collected by a separate medical device. As one nonlimiting example, the breathing pattern characteristic may indicate that the patient is breathing too fast, while the cardiac activity component of the CA signals also indicates that the heart rate is unduly fast. The combination of characteristics could be indicative of various non-physiologic episodes being experienced by the patient including, but not limited to, a heart attack. In response to detection of an undesirable breathing pattern, methods and devices herein may undertake various actions. For example, an implantable medical device and/or a portable non-lead based wearable device may wirelessly communicate an alarm indicative of the breathing pattern to an external local or remote device. For example, an alarm and be transmitted to a first responder system or other medical network to request an ambulance be dispatched. As another example, when the patient is in a hospital, clinic, senior or assisted care living facility, the alarm may be conveyed to a central desk within the facility to inform the staff that a patient at the facility is in need of immediate attention.

In accordance with embodiments herein, a medical device is provided. The medical device includes a sensing circuitry configured to obtain cardiac activity (CA) signals indicative of cardiac activity over one or more beats. The medical device includes a filter configured to separate, from the CA signals, a respiratory component that varies based on at least one of respiration rate or respiration depth. The medical device includes memory that is configured to store program instructions. The medical device includes a processor that, when executing the program instructions, is configured to analyze the respiratory component to identify a respiration characteristic of interest (COI). The respiration COI is based on at least one of variations in an amplitude of the respiratory component or an interval within the respiratory component and identifies a respiration anomaly based on the respiration COI.

Optionally, the processor may be configured to analyze the respiratory component for the respiration COI identify at least one of a respiration rate, a respiration depth, or respiration irregularity, that may be indicative of at least one of hypopnea, sleep apnea, dyspnea, tachypnea, bradypnea. The processor may be configured to identify the respiration anomaly to be i) sleep apnea when the interval within the respiration component drops below an interval threshold, or ii) hypopnea when the amplitude of the respiration component falls below an amplitude threshold. The filter may represent at least one of a band pass filter or a low-pass filter configured to separate the respiratory component from a cardiac activity component within the CA signals. Filter blocks signal components may have a frequency of greater than 1 Hz.

Optionally, the filter may represent a band pass filter that removes analog-to-digital converter (ADC) baseline component to avoid baseline wandering within the respiration component. The processor may be further configured to determine interval within the respiration component by counting a number of at least one of peaks or valleys in the respiratory component over a period of time. The processor may be configured to analyze the respiration component for at least one of an area under the curve, a slope, amplitude or intervals between peaks or valleys in connection with identifying the respiration COI. The medical device may be an implantable and further comprises electrodes electrically connected to the sensing circuit, the electrodes defining a sensing vector along which the CA signals are sensed. The interval within the signal component may correspond to a breathing cycle as indicated by a period between successive peaks or valleys of the signal component.

Optionally, the processor may be configured to at least one of perform an action or provide an output, including at least one of: a) adjusting parameters of an implantable medical device; b) initiating an operation to collect additional patient data, from the same device or from another device; c) at least one of delivering or changing a therapy delivered by an external device or the medical device; d) delivering or changing a drug regiment or dosage; e) automatically scheduling a patient-physician appointment; f) scheduling a follow-up diagnostic procedure; g) providing an output indicating that a patient is in immediate need of medical assistance; h) providing an output request to automatically dispatching an ambulance or other first responder to the patient; i) providing an output indicating a change in a patient's condition; j) providing an output indicating a patient is experiencing at least one apnea, a panic attack, hyperventilating, heart attack, has passed out, or a seizure; or k) tracking apnea burden over time.

In accordance with embodiments herein, a method is provided. The method comprises filtering cardiac activity (CA) signals indicative of cardiac activity over one or more beats to separate a respiratory component that varies based on at least one of respiration rate or respiration depth. The method analyzes the respiratory component to identify a respiration characteristic of interest (COI). The respiration COI is based on at least one of variations in an amplitude of the respiratory component or an interval within the respiratory component. The method identifies a respiration anomaly based on the respiration COI.

Optionally, the method may analyze the respiratory component for the respiration COI to identify at least one of a respiration rate, a respiration depth, or respiration irregularity, that is indicative of at least one of hypopnea, sleep apnea, dyspnea, tachypnea, or bradypnea. The method may identify the respiration anomaly to be i) sleep apnea when the interval within the respiration component drops below an interval threshold, or ii) hypopnea when the amplitude of the respiration component falls below an amplitude threshold. The filtering may include applying at least one of a band pass filter or a low-pass filter to separate the respiratory component from a cardiac activity component within the CA signals, wherein filtering blocks signal components having a frequency of greater than 1 Hz.

Optionally, the filtering may include applying a band pass filter that removes ADC baseline component to avoid baseline wandering within the respiration component. The method may determine an interval within the respiration component by counting a number of at least one of peaks or valleys in the respiratory component over a period of time. The method may analyze the respiration component for at least one of an area under the curve, a slope, amplitude or intervals between peaks or valleys in connection with identifying the respiration COI. The interval within the signal component may correspond to a breathing cycle as indicated by a period between successive peaks or valleys of the signal component.

Optionally, the method may comprise at least one of performing an action or providing an output, including at least one of: a) adjusting parameters of an implantable medical device; b) initiating an operation to collect additional patient data, from the same device or from another device; c) at least one of delivering or changing a therapy delivered by an external device or the medical device; d) delivering or changing a drug regiment or dosage; e) automatically scheduling a patient-physician appointment; f) scheduling a follow-up diagnostic procedure; g) providing an output indicating that a patient is in immediate need of medical assistance; h) providing an output request to automatically dispatching an ambulance or other first responder to the patient; i) providing an output indicating a change in a patient's condition; or j) providing an output indicating a patient is experiencing at least one apnea, a panic attack, hyperventilating, heart attack, has passed out, or a seizure.

Optionally, the method may comprise comparing non-CA signals indicative of at least one of patient posture or patient activity to a threshold and based on the comparing, initiating obtaining of the CA signals. In addition, or alternatively, the method may optionally comprise analyzing non-CA signals indicative of at least one of patient posture or patient activity for an activity COI, and identifying a sleep behavior pattern based on the activity COI and the respiration COI. In addition, or alternatively, the method may optionally comprise combining non-CA signals indicative of at least one of patient posture or patient activity with the respiration COI over a period of time to define a trend in sleep quality

### BRIEF DESCRIPTION OF THE DRAWINGS

Figure 1 illustrates an IMD and external device implanted proximate to a heart in a patient and implemented in accordance with one embodiment.
Figure 2A illustrates an example block diagram of an IMD that is implanted into the patient as part of the implantable cardiac system.
Figure 2B illustrates a sensing and filtering circuit implemented in accordance with embodiments herein.
Figure 2C illustrates a graph for a transfer function representative of a passband that may be utilized with the filter in accordance with embodiments herein.
Figure 3 illustrates an example of CA signals collected for a series of heartbeats by electrodes that define a corresponding sensing vector.
Figure 4 illustrates an example of the respiratory component separated from the CA signals of Figure 3 in accordance with embodiments herein.
Figure 5 illustrates a process for detecting respiratory anomalies based on the respiratory component within an electrical cardiac activity signal in accordance with embodiments herein.
Figure 6 illustrates a process for collecting baseline information to be utilized subsequently for analyzing respiratory components for respiration anomalies in accordance with embodiments herein.
Figure 7 illustrates a block diagram of a system for integrating external diagnostics with remote monitoring of data provided by implantable medical devices in accordance with embodiments herein.
Figure 8 illustrates a high-level flowchart of a method, implemented by a medical network, for processing respiration anomalies in connection with other medical devices that collect BGA data and/or IMD data in accordance with embodiments herein.
Figure 9 illustrates a healthcare system formed in accordance with embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The terms "cardiac activity signal" and "CA signal" shall refer to electrical signals that are indicative of cardiac activity and are collected by implantable electrodes and/or surface electrodes provided with a portable non-lead based wearable device. The CA signals may be IEGM signals from an IMD and/or ECG signals from a non-lead based wearable device. For the avoidance of doubt, the term CA signal shall not include an ECG signal collected by a 12 lead ECG monitoring system, nor a Holter monitor that utilizes 3 or more wire-based leads.

The term "non-lead based wearable device" shall refer to battery-powered mobile electronic devices that are worn or carried by the patient while a patient is mobile, where the electronic device is coupled to sensing electrodes that are either integrated into the housing of the electronic device or otherwise in close proximity thereto, in a non-lead based manner. For example, the electrodes may be physically separate from the device housing but configured to wirelessly communicate with the device. For the avoidance of doubt, the term non-lead based wearable device shall not mean and shall not include a 12 lead ECG monitoring system, nor a Holter monitor that utilizes 3 or more wire-based leads.

The term "non-CA signals" shall mean signals other than IEGM or ECG signals.

The term "tidal volume" shall mean the lung volume representing the normal volume of air displaced between normal inhalation and exhalation when extra effort is not applied. By way of example, a healthy, young human adult, tidal volume is approximately 500 mL per inspiration or 7 mL/kg of body mass.

The terms "body generated analyte" and "BGA" shall mean a test substance or specimen that is naturally generated by or naturally present in a human body. The test substance or specimen may be in liquid form (e.g., blood or other bodily fluid), solid form (e.g., tissue, fat, muscle, bone, or other organ-based material), gas form, cellular form or otherwise.

The term "BGA test device" shall mean any and all equipment, devices, disposable products utilized to collect and analyze a BGA. The BGA test device may implement one or more of the methods, devices and systems described herein and/or in one or more of the patents, published applications or other publications referenced herein.

The term "IMD data" shall mean any and all types of information and signals conveyed from an implantable medical device to a local or remote external device. Nonlimiting examples of IMD data include cardiac activity signals (e.g., intracardiac electrogram or IEGM signals), respiration data (e.g. respiration components, respiration COI, breathing anomalies), impedance signals (e.g., cardiac, pulmonary or transthoracic impedances), accelerometer signatures (e.g., activity signals, posture/orientation signals, heart sounds), pulmonary arterial pressure signals, MCS rpm levels, MCS flow rates, device alerts and the like.

The terms "patient data entry device" and "PDE device" shall mean an electronic device that includes a user interface that is configured 1) to receive patient data that is entered by the patient and/or 2) to receive patient data in connection with actions/decisions by the patient. A PDE device is different from an IMD and a BGA test device. The PDE device is configured to receive behavior related medical data that differs from IMD data and that differs from BGA data. The PDE devices may include, but are not limited to, smart phones, desktop or laptop computers, tablet devices, smart TVs, fixed cameras, smart watch, wearable heart rate monitor, portable or handheld cameras, recording devices, digital personal assistant (DPA) devices and the like. One nonlimiting example of a PDE device is a smart phone implementing the "HEMAAPP" application, developed at the University of Washington. Another example is a smart phone application developed by Wilbur Lam at the Aflac Cancer and Blood Disorders Center of Children's Healthcare of Atlanta, and Wallace Coulter, a faculty member in the Department of biomedical engineering at Georgia Tech. The PDE device may include an electronic device sold under the trademark ALEXA^{®} by Amazon.com Inc., and/or an electronic device sold under the trademark NOW^{®} by Google LLC., and the like. In addition, the PDE devices may represent various types of devices configured to record audio and/or voice signatures, detect gestures and movements and the like. The PDE device may include a graphical user interface, through which the patient or another user enters the patient data. Optionally, the PDE device may include audio and/or video sensors/cameras that may receive patient data. For example, a user may use a keyboard, touch screen and/or mouse to enter patient data. Optionally, the user may enter the patient data through spoken words (e.g., "Alexa I just took my medication", "Alexa I am eating 3 slices of peperoni pizza", "Alexa I am eating an apple", "Alexa I am drinking a 12 oz. soda and eating a candy bar). Optionally, the PDE device may automatically track actions by a patient, such as through the use of cameras to visually watch a patients actions, through the use of microphones to "listen" to a patient's actions, and/or through the use of other types of sensors (e.g., refrigerator or kitchen cabinet door sensor, sensor on a treadmill). For example, a camera may capture video that is processed by a processor utilizing image recognition to identify what a patient is eating/drinking, when the patient eats/drinks, and how much the patient consumed. Optionally, the BRM device may include a position tracking device sold under the trademark FITBIT^{®} by Fitbit Inc. or other types of position tracking devices. The position tracking device may monitor and collect, as BRM data, movement information, such as a number of steps or distance traveled in a select period of time, a rate of speed, a level of exercise and the like. Optionally, the BRM device may monitor and collect, as BRM data, heart rate.

Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System".

Additionally or alternatively, the IMD may be a leadless implantable medical device (LIMD) that include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

Additionally or alternatively, the IMD may be a subcutaneous IMD that includes one or more structural and/or functional aspects of the device(s) described in U.S. Patent Application 2019/0336753, titled "Subcutaneous Implantation Medical Device With Multiple Parasternal-Anterior Electrodes"; U.S. Patent 10,722,704, titled "Implantable Medical Systems And Methods Including Pulse Generators And Leads"; U.S. Patent Application 2019/0336747, titled "Single Site Implantation Methods For Medical Devices Having Multiple Leads". Further, one or more combinations of IMDs may be utilized from the above patents and applications in accordance with embodiments herein.

Additionally or alternatively, the IMD may be a leadless cardiac monitor (ICM) that includes one or more structural and/or functional aspects of the device(s) described in U.S. Patent 9,949,660, entitled, "METHOD AND SYSTEM TO DISCRIMINATE RHYTHM PATTERNS IN CARDIAC ACTIVITY".

Embodiments may be implemented in connection with one or more PIMDs. Non-limiting examples of PIMDs may include passive wireless sensors used by themselves, or incorporated into or used in conjunction with other implantable medical devices (IMDs) such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, the PIMD may include one or more structural and/or functional aspects of the device(s) described in U.S. Patent No. 9,265,428 entitled "Implantable Wireless Sensor", U.S. Patent No. 8,278,941 entitled "Strain Monitoring System and Apparatus", U.S. Patent No. 8,026,729 entitled "System and Apparatus for In-Vivo Assessment of Relative Position of an Implant", U.S. Patent No. 8,870,787 entitled "Ventricular Shunt System and Method", and U.S. Patent No. 9,653,926 entitled "Physical Property Sensor with Active Electronic Circuit and Wireless Power and Data Transmission".

Additionally or alternatively, embodiments herein may be implemented in connection with an arrhythmia confirmation process such as described in: U.S. Patent No. 10,729,346, titled "METHOD AND SYSTEM FOR SECOND PASS CONFIRMATION OF DETECTED CARDIAC ARRHYTHMIC PATTERNS"; U.S. Patent Application 2019/0336026, titled "METHOD AND SYSTEM TO DETECT R-WAVES IN CARDIAC ARRHYTHMIC PATTERNS"; U.S. Patent No. 10,874,322, titled "METHOD AND SYSTEM TO DETECT POST VENTRICULAR CONTRACTIONS IN CARDIAC ARRHYTHMIC PATTERNS"; and U.S. Patent No. 10,777,880, titled "METHOD AND SYSTEM TO DETECT NOISE IN CARDIAC ARRHYTHMIC PATTERNS".

Additionally or alternatively, embodiments herein in connection with an integrated healthcare patient management system or network, such as described in U.S. Patent Application 2021/0020294, titled "METHODS, DEVICE AND SYSTEMS FOR HOLISTIC INTEGRATED HEALTHCARE PATIENT MANAGEMENT".

Additionally or alternatively, embodiments herein may be implemented in connection with the methods and systems described in "METHOD AND SYSTEM FOR HEART CONDITION DETECTION USING AN ACCELEROMETER", U.S. Provisional Application No. 63/021,775 filed on 8 August 2020.

Additionally or alternatively, embodiments herein may be implemented in connection with the methods and systems described in "SYSTEM FOR VERIFYING A PATHOLOGIC EPISODE USING AN ACCELEROMETER", U.S. Provisional Application Nos. 63/021,778 filed on 8 May 2020 and 63/139,304 filed on 19 January 2021.

While some embodiments are described in connection with an IMD coupled to a transvenous lead, it is understood that the present improvements are not so limited. Instead, embodiments herein may be implemented in connection with IMDs that do not utilize transvenous leads, such as IMDs with subcutaneous leads, implantable cardiac monitors, leadless therapy devices and the like.

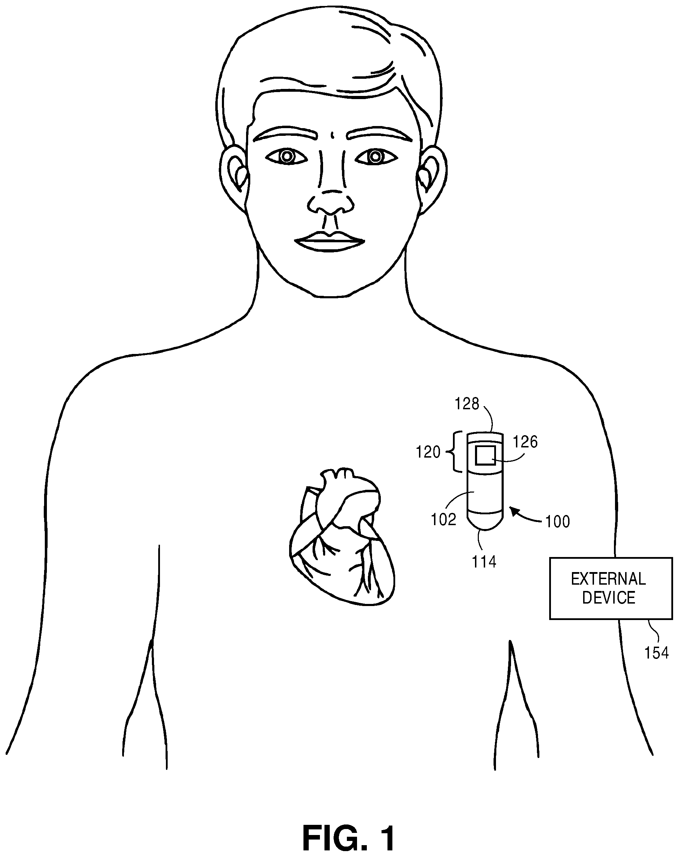

Figure 1 illustrates an implantable medical device (IMD) 100 intended for subcutaneous implantation at a site near the heart. The IMD 100 includes a pair of spaced-apart sense electrodes 114, 126 positioned with respect to a housing 102. The sense electrodes 114, 126 provide for detection of far field electrogram signals. Numerous configurations of electrode arrangements are possible. For example, the electrode 114 may be located on a distal end of the IMD 100, while the electrode 126 is located on a proximal side of the IMD 100. Additionally or alternatively, electrodes 126 may be located on opposite sides of the IMD 100, opposite ends or elsewhere. The distal electrode 114 may be formed as part of the housing 102, for example, by coating all but a portion of the housing with a nonconductive material such that the uncoated portion forms the electrode 114. In this case, the electrode 126 may be electrically isolated from the housing 102 electrode by placing it on a component separate from the housing 102, such as the header 120. Optionally, the header 120 may be formed as an integral portion of the housing 102. The header 120 includes an antenna 128 and the electrode 126. The antenna 128 is configured to wirelessly communicate with an external device 154 in accordance with one or more predetermined wireless protocols (e.g., Bluetooth, Bluetooth low energy, Wi-Fi, etc.).

The housing 102 includes various other components such as: sense electronics for receiving signals from the electrodes, a microprocessor for analyzing the far field CA signals, including assessing the presence of R-waves in cardiac beats occurring while the IMD is in different IMD locations relative to gravitational force, a loop memory for temporary storage of CA data, a device memory for long-term storage of CA data, sensors for detecting patient activity, including an accelerometer for detecting acceleration signatures indicative of heart sound, and a battery for powering components.

In at least some embodiments, the IMD 100 is configured to be placed subcutaneously utilizing a minimally invasive approach. Subcutaneous electrodes are provided on the housing 102 to simplify the implant procedure and eliminate a need for a transvenous lead system. The sensing electrodes may be located on opposite sides of the device and designed to provide robust episode detection through consistent contact at a sensor-tissue interface. The IMD 100 may be configured to be activated by the patient or automatically activated, in connection with recording subcutaneous ECG signals.

The IMD 100 senses far field, subcutaneous CA signals, processes the CA signals to detect arrhythmias and if an arrhythmia is detected, automatically records the CA signals in memory for subsequent transmission to an external device 154. The IMD 100 includes a filter configured to separate, from the CA signals, a respiratory component that varies based on at least one of respiration rate or respiration depth. The IMD analyzes the respiratory component to identify a respiration characteristic of interest (COI). The respiration COI is based on at least one of variations in an amplitude of the respiratory component or an interval within the respiratory component. The IMD identifies a respiration anomaly based on the respiration COI. For example, the IMD analyzes the respiratory component for the respiration COI that identifies at least one of a respiration rate or a respiration depth, that is indicative of at least one of hypopnea, sleep apnea, dyspnea, (difficult or labored breathing), tachypnea (rapid breathing), or bradypnea.

Optionally, the IMD may be configured to only separate the respiration component from the CA signals at certain times, such as only when certain activity occurs, or the patient is in certain posture requirements.

The IMD 100 is implanted in a position and orientation such that, when the patient stands, the IMD 100 is located at a reference position and orientation with respect to a global coordinate system 10 that is defined relative to a gravitational direction 12. For example, the gravitational direction 12 is along the Z-axis while the X-axis is between the left and right arms.

As explained herein, the IMD 100 includes electrodes that collect cardiac activity (CA) signals in connection with multiple cardiac beats and in connection with different IMD locations (e.g., different positions and/or different orientations). The IMD may change location within a subcutaneous pocket relative to an initial implant position through translation and/or rotation, such as i) moving up and down (elevating/heaving) within the subcutaneous pocket; ii) moving left and right (strafing/swaying); iii) moving forward and backward (walking/surging); iv) swiveling left and right (yawing); v) tilting forward and backward (pitching); and pivoting side to side (rolling). The IMD 100 also includes one or more sensors to collect device location information indicative of movement of the IMD 100 along one or more degrees of freedom, namely translational motion along X, Y, and Z directions, and/or rotationally motion along pitch, yaw and/or roll directions.

### Implantable Medical Device

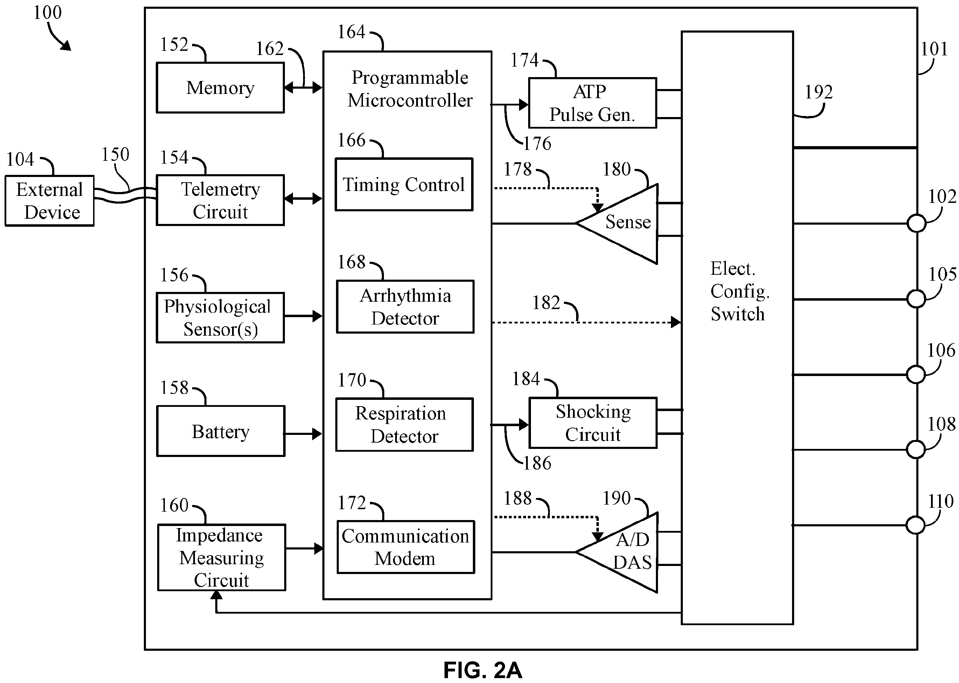

Figure 2A illustrates an example block diagram of an IMD 100 that is implanted into the patient as part of the implantable cardiac system. The IMD 100 may be implemented to monitor ventricular activity alone, or both ventricular and atrial activity through sensing circuit. The IMD 100 has a housing 102 to hold the electronic/computing components. The housing 102 (which is often referred to as the "can," "case," "encasing," or "case electrode") may be programmably selected to act as an electrode for certain sensing modes. Housing 102 further includes a connector (not shown) with at least one terminal 112 and optionally an additional terminal 115. The terminals 112, 115 may be coupled to sensing electrodes that are provided upon or immediately adjacent the housing 102. Optionally, more than two terminals 112, 115 may be provided in order to support more than two sensing electrodes, such as for a bipolar sensing scheme that uses the housing 102 as a reference electrode. Additionally or alternatively, the terminals 112, 115 may be connected to one or more leads having one or more electrodes provided thereon, where the electrodes are located in various locations about the heart. The type and location of each electrode may vary.

The IMD 100 includes a programmable microcontroller 164 that controls various operations of the IMD 100, including cardiac monitoring and, optionally, stimulation therapy. Microcontroller 164 includes a microprocessor (or equivalent control circuitry), RAM and/or ROM memory, logic and timing circuitry, state machine circuitry, and I/O circuitry. While not shown, the IMD 100 may further includes a first chamber pulse generator 174 that generates stimulation pulses for delivery by one or more electrodes coupled thereto.

When the IMD 100 is configured to deliver therapy, the microcontroller 164 may include timing control circuitry 166 to control the timing of the stimulation pulses (e.g., pacing rate, atrio-ventricular (AV) delay, atrial interconduction (A-A) delay, or ventricular interconduction (V-V) delay, etc.). The timing control circuitry 166 may also be used for the timing of refractory periods, blanking intervals, noise detection windows, evoked response windows, alert intervals, marker channel timing, and so on. Microcontroller 164 also has an arrhythmia detector 168 for detecting arrhythmia conditions and a respiration detector 170 to review and analyze one or more features of respiration components, respiration COI and respiration anomalies. Although not shown, the microcontroller 164 may further include other dedicated circuitry and/or firmware/software components that assist in monitoring various conditions of the patient's heart and managing pacing therapies.

The respiration detector 170 is configured to analyze the respiratory component to identify a respiration characteristic of interest (COI), wherein the respiration COI is based on at least one of variations in an amplitude of the respiratory component or an interval within the respiratory component. The respiration detector 170 is further configured to identify a respiration anomaly based on the respiration COI. Additionally or alternatively, the respiration detector 170 may further analyze the respiratory component for the respiration COI that identifies at least one of a respiration rate or a respiration depth, that is indicative of at least one of hypopnea, sleep apnea, dyspnea, (difficult or labored breathing), tachypnea (rapid breathing), or bradypnea. Additionally or alternatively, the respiration detector 170 may further identify the respiration anomaly to be i) sleep apnea when the interval within the respiration component drops below an interval threshold, ii) hypopnea when the amplitude of the respiration component falls below an amplitude threshold, iii) dyspnea when the amplitude drops below an interval threshold and stays below the threshold for a longer period of time compared to sleep apnea, or iv) tachypnea based on a number of breaths greater than a normal range. Additionally or alternatively, the respiration detector 170 may further determine an interval within the respiration component by counting a number of at least one of peaks or valleys in the respiratory component over a period of time. For example, the respiration detector 170 may analyze the respiration component for at least one of an area under the curve, a slope, amplitude or intervals between peaks or valleys in connection with identifying the respiration COI. As explained herein, the interval within the signal component may correspond to a breathing cycle as indicated by a period between successive peaks or valleys of the signal component.

Additionally or alternatively, the respiration detector 170 may at least one of perform an action or provide an output, including at least one of: a) adjusting parameters of an implantable medical device, b) initiating an operation to collect additional patient data, from the same device or from another device, c) at least one of delivering or changing a therapy delivered by an external device or the medical device, d) delivering or changing a drug regiment or dosage, e) automatically scheduling a patient-physician appointment, f) scheduling a follow-up diagnostic procedure, g) providing an output indicating that a patient is in immediate need of medical assistance, h) providing an output request to automatically dispatching an ambulance or other first responder to the patient, i) providing an output indicating a change in a patient's condition, j) providing an output indicating a patient is experiencing at least one apnea, a panic attack, hyperventilating, heart attack, has passed out, or a seizure, or k) tracking apnea burden over time (e.g., tracking a number of apnea events that occur in a time interval, such as an hour). For example, the apnea burden may be tracking in combination with information recorded and tracked by a CPAP system. Additionally or alternatively, the respiration detector 170 may be further configured to obtain non-CA signals indicative of at least one of patient posture or patient activity; and utilizing the non-CA signals in combination with the respiratory component as follows: a) comparing the non-CA signals to a threshold and based on the comparing initiating an analysis of the CA signals (e.g., CA signals would generally be obtained constantly); b) analyzing the non-CA signals for an activity COI, and identifying a sleep behavior pattern based on the activity COI and the respiration COI; and/or c) combining the non-CA signals with the respiration COI over a period of time to define a trend in sleep quality.

Optionally, the respiration detector 170 may be inactive for periods of time and activated only when certain criteria are present, such as then certain activity occurs and/or when a patient meets certain posture requirements.

The IMD 100 is further equipped with a communication modem (modulator/demodulator) 172 to enable wireless communication with other devices, implanted devices and/or external devices. The IMD 100 includes sensing circuitry 180 selectively coupled to one or more electrodes that perform sensing operations, through the switch 192, to detect the presence of cardiac activity. The sensing circuitry 180 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 192 determines the sensing polarity of the cardiac signal by selectively closing the appropriate switches. In this way, the clinician may program the sensing polarity independent of the stimulation polarity.

When utilized in an IMD configured to deliver therapy (e.g., in a subcutaneous IMD), the output of the sensing circuitry 180 may be utilized by the microcontroller 164 to trigger or inhibit the pulse generator in response to the absence or presence of cardiac activity. The sensing circuitry 180 receives a control signal 178 from the microcontroller 164 for purposes of controlling the gain, threshold, polarization charge removal circuitry (not shown), and the timing of any blocking circuitry (not shown) coupled to the inputs of the sensing circuitry.

In the example of Figure 1, a single sensing circuit 180 is illustrated. Optionally, the IMD 100 may include multiple sensing circuit, similar to sensing circuit 180, where each sensing circuit is coupled to one or more electrodes and controlled by the microcontroller 164 to sense electrical activity detected at the corresponding one or more electrodes. The sensing circuit 180 may operate in a unipolar sensing configuration or in a bipolar sensing configuration.

The IMD 100 further includes an analog-to-digital (A/D) data acquisition system (DAS) 190 coupled to one or more electrodes via the switch 192 to sample cardiac signals across any pair of desired electrodes. The data acquisition system 190 is configured to acquire intracardiac electrogram signals, convert the raw analog data into digital data, and store the digital data for later processing and/or telemetric transmission to an external device 104 (e.g., a programmer, local transceiver, or a diagnostic system analyzer). The data acquisition system 190 is controlled by a control signal 188 from the microcontroller 164.

The microcontroller 164 is coupled to a memory 152 by a suitable data/address bus 162. The programmable operating parameters used by the microcontroller 164 are stored in memory 152 and used to customize the operation of the IMD 100 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The telemetry circuit 154 allows intracardiac electrograms and status information relating to the operation of the IMD 100 (as contained in the microcontroller 164 or memory 152) to be sent to the external device 104 through the established communication link 150.

The IMD 100 can further include one or more physiologic sensors 156. Such sensors are commonly referred to as "rate-responsive" sensors because they are typically used to adjust pacing stimulation rates according to the activity state of the patient. However, the physiological sensor 156 may further be used to detect changes in cardiac output, changes in the physiological condition of the heart, or diurnal changes in activity (e.g., detecting sleep and wake states). The physiologic sensor 156 may also be utilized to detect patient posture. As described herein, patient posture and/or patient activity information may be utilized in connection with the determination of a respiration anomaly, such as in connection with tracking progression and trends in sleep patterns, a physiologic condition, progression of heart failure, and the like. Additionally or alternatively, the patient posture and/or patient activity information may be utilized in connection with the respiration anomaly for more acute matters, such as detecting a heart attack, anxiety attack and the like.

A battery 158 provides operating power to all of the components in the IMD 100. The IMD 100 further includes an impedance measuring circuit 160, which can be used for many things, including: lead impedance surveillance during the acute and chronic phases for proper lead positioning or dislodgement; detecting operable electrodes and automatically switching to an operable pair if dislodgement occurs; measuring thoracic impedance for determining shock thresholds; detecting when the device has been implanted; measuring stroke volume; and detecting the opening of heart valves; and so forth. The impedance measuring circuit 160 is coupled to the switch 192 so that any desired electrode may be used. While not shown, optionally, the IMD 100 can be operated as an implantable cardioverter/defibrillator (ICD) device, which detects the occurrence of an arrhythmia and automatically applies an appropriate electrical shock therapy to the heart aimed at terminating the detected arrhythmia. To this end, the microcontroller 164 would further control a shocking circuit.

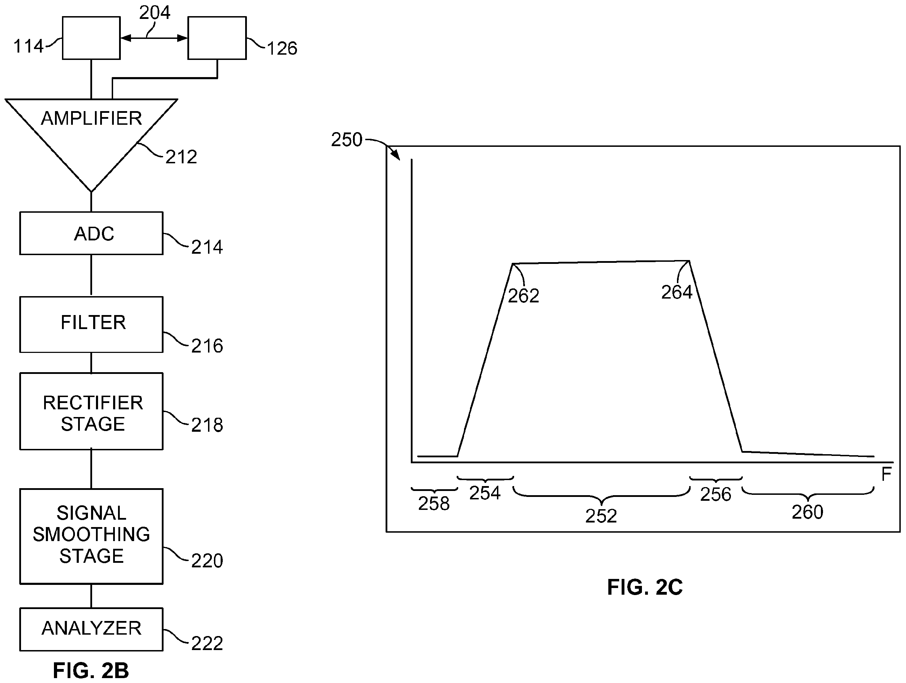

Figure 2B illustrates a sensing and filtering circuit implemented in accordance with embodiments herein. By way of example, the sensing filtering circuit may be implemented within the sensing circuit 180 (Figure 2A) and/or in connection with the A/D DAS 190. Electrodes 114, 126 define a sensing vector 204 there between. The electrodes 114, 1216 are coupled to an amplifier 212, an output of which is supplied to an analog to digital (A/D) converter 214 (such as A/D 190 in Figure 2A). The A/D converter 214 converts the signal to a digital signal that is output to the filter 216. The filter 216 is configured to separate the respiration component from the cardiac activity component within the CA signals.

The filter 216 blocks signal components having a frequency greater than a predetermined upper cut off frequency. The filter 216 may be configured as a low-pass filter or a band pass filter having an upper limit for the passband that is at a relatively low frequency. For example, an upper cut off frequency for the passband of the filter may be at approximately 1.0 Hz, or more preferably at 0.5 hertz or even more preferably at 0.2 Hz, or even more preferably at 0.1 Hz. Additionally or alternatively, the filter 216 may be constructed as a band pass filter with an upper limit as noted above in connection with the low-pass filter. The band pass filter may also be configured to have a lower cut off frequency configured to remove any DC bias component from the respiration component, such as to prevent a baseline wander in which a baseline signal continuously fluctuates which similarly causes peaks and valleys to fluctuate based on factors other than respiration.

The respiratory component output by the filter 216 may optionally be supplied to a rectifier 218, the output of which may be supplied to a signal smoothing stage 220. The output of the signal smoothing stage 220 may then be provided to an analyzer module 222. Optionally, the rectifier 218 and signal smoothing stage 220 may be omitted entirely and the respiratory component output of the filter 216 provided directly to the analyzer module 222. By way of example, the analyzer module 222 may represent a processor, such as the programmable microcontroller 164 (Figure 2A), configured to execute program instructions to perform the various analyses, provide the outputs and take other actions as described herein.

Based on the determination by the analyzer module 222, an output may include triggering an alarm. For example, the alarm may provide an audible, vibratory or other output detectable to the patient. For example, when the alarm is provided within an implantable device, the alarm may vibrate or produce another perceptible output intended to wake up or otherwise alert the patient. Additionally or alternatively, the alarm may represent an application operating on a local external device, such as a tablet device, smart phone or other external device that may be located in a bedroom or other location proximate to where patient may experience the breathing anomaly of interest. For example, in connection with sleep apnea, the patient may lay a smart phone next to the bed at night. When sleep apnea is detected by the analyzer 222, an alert is wirelessly transmitted (e.g. through a BLE, Bluetooth or other wireless communications connection) to the local external device, which in turn generates an audible or other perceptible alarm to wake up the patient.

Figure 2C illustrates a graph 250 for a transfer function representative of a passband that may be utilized with the filter 214. The transfer function illustrates the magnitude of the frequency along the horizontal axis and amplitude along the vertical axis. The transfer function has a passband 252 between a lower transition region 254 and an upper transition region 256. The lower transition region 254 is preceded by a stopband 258, while the upper transition region 256 is followed by a stopband 260. The passband 252 is bordered by lower and upper cutoff frequencies 262 and 264. By way of example, the filter 212 may exhibit a passband corresponding to the transfer function.

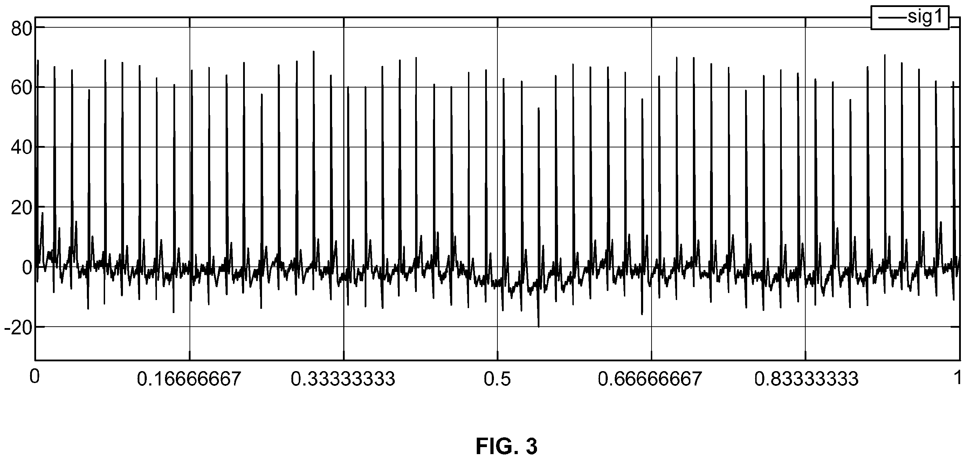

Figure 3 illustrates an example of CA signals collected for a series of heartbeats by electrodes that define a corresponding sensing vector. As explained herein, the CA signals are passed through a filter that separates a respiratory component from other components within the CA signals.

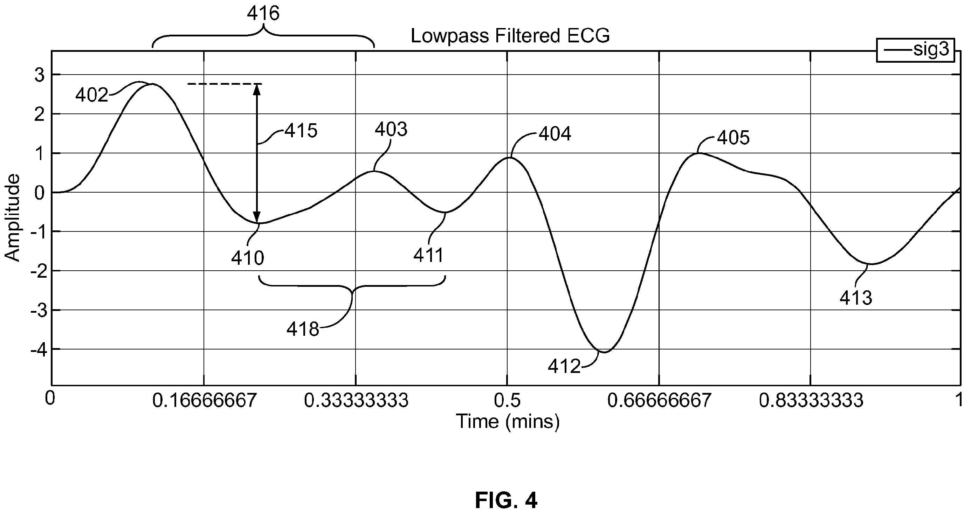

Figure 4 illustrates an example of the respiratory component separated from the CA signals of Figure 3 in accordance with embodiments herein. As evident from the comparison of Figures 3 and 4, the respiratory component exhibits a much lower frequency with substantially smaller amplitude variations (dynamic range) between peaks and valleys, as compared to the frequency components and amplitude dynamic range of the CA signals. The respiratory component includes peaks 402 - 405 and valleys 410 - 413 which correspond to points in which the patient inhales and exhales. For example, the peaks 402 - 405 may correspond to points in breathing cycles were a patient has completed inspiration, while the points 410 - 413 correspond to points in breathing cycles were a patient has completed expiration.

The levels for the peaks and valleys 402 - 405 and 410 - 413 are also representative of a depth or degree of the corresponding inspiration or expiration. For example, the peak 402 may correspond to a point in a normal breathing cycle where a patient has completed a normal, full inspiration, while the valley 412 may correspond to a point a normal breathing cycle were patient has completed a normal, full expiration. The lower level peaks and valleys 403 - 405, 410, 411 and 413 correspond to points in a breathing cycle were patient has completed inspiration and expiration but has not undergone a full breath. The distance 415 between successive peaks and valleys, such as between peak 402 and valley 410, represents the volume of air taken in by the patient during the corresponding breath. During a normal breathing cycle, the distance 415 would correspond to the patient's tidal volume.

The amplitude of the respiratory component of interest exhibits relatively little fluctuation between peaks and valleys, depending on several factors such as electrode placement, electrode conductivity, electrode spacing, among other factors. For example, the variation in the respiratory component amplitude may vary over a range of 0.1 mV to 5 mV, or more specifically between 0.1 mV and 2.5 mV, and even more specifically between 0.1 mV and 0.5mV.

In connection with embodiments herein, the respiratory component of Figure 4 is analyzed to identify the respiration COI. In the example of figure 4, the respiration COI corresponds to the peaks and valleys 402 - 405 and 410 - 413. The respiration COI is then further analyzed to identify the respiration anomaly. For example, the interval between successive peaks may be analyzed, such as the interval 416 between peaks 402 and 403. Additionally or alternatively, the interval between valleys may be analyzed, such as the interval 418 between valleys 410 and 411. Additionally or alternatively, the respiration COI may correspond to the amplitude/level for the peaks and valleys, an area under the curve or other similar characteristics.

When a patient experiences apnea or hypopnea, the interval for the peaks or valleys will lengthen as breathing has either ceased (apnea) or became too reduced (hypopnea) to be detected. In accordance with embodiments herein, sleep apnea may be identified based on the lengthen the in the breathing interval.

### Process to Detect Respiratory Anomalies

Figure 5 illustrates a process for detecting respiratory anomalies based on the respiratory component within an electrical cardiac activity signal in accordance with embodiments herein. It should be recognized that the CA signals may be continuously collected and analyzed for various reasons such as to identify arrhythmias, deliver therapies and the like, as described in the various patents and published applications described herein. The process of Figure 5 may be performed continuously or periodically each and every time CA signals are collected.

Additionally or alternatively, the process of Figure 5 may be performed only at select times in response to certain criteria. For example, in accordance with new and unique aspects herein, the CA signals (e.g., IEGM/ECG) may be collected when patients are inactive or asleep to minimize the effect that activity or posture has on the CA signals. It has been found that posture can impact IEGM signal amplitude and therefore managing when to perform the operations of Figure 5 relative to posture can be important. Embodiments herein utilize 3D accelerometer signals to monitor activity level as well as posture measurements. One or more processors of the IMD may determine that the activity level has dropped below a lower threshold and remained below the lower threshold for a select period of time, thereby indicating that the patient is asleep. Additionally or alternatively, the one or more processors may determine that the patient posture is supine and has remained supine for a select period of time, as another indicator that the patient is asleep. Based on one or both of the activity level and/or posture, the one or more processors may determine that the time is appropriate for the process of Figure 5 to be implemented to filter respiration components from the IEGM/ECG signals and utilize the respiration components to identify respiratory anomalies.

At optional step 502, a sensing circuit collects electrical CA signals indicative of cardiac activity over one or more beats. The CA signals are collected over one or more sensing vectors that are defined by a combination of two or more sensing electrodes. The sensing vector and configuration of electrodes may vary based upon the system implementing the process. The electrode configuration may be implemented in various manners in connection with the various types of external and implantable devices described herein. For example, the electrodes may be located on an intravenous lead positioned within or proximate the heart. Additionally or alternatively, the electrodes may be held on a housing of a leadless implantable medical device that is entirely located within the chamber of the heart or within a vessel proximate the heart. Additionally or alternatively, the electrodes may be held on a housing of an implantable cardiac monitor located remote from the heart, but implanted subcutaneously. Additionally or alternatively, the electrodes may be provided on a lead that is implanted subcutaneously, but not within the heart, such as utilized with subcutaneous ICDs and the like. Additionally or alternatively, the electrodes may be provided on a neural stimulation lead located proximate to a region of the nervous system, such as within or proximate to the spine, brainstem and the like.

Additionally or alternatively, the electrode configuration may be provided on a leadless pacemaker or other leadless implantable device located within or proximate a chamber of the heart. The electrode configuration defines a sensing vector between the electrodes that may be configured to perform far field sensing for cardiac activity in the chamber of the heart in which the device is located and/or in a remote chamber of the heart remote from where the devices implanted.

Additionally or alternatively, the electrode configuration may be provided on an implantable cardiac monitor that is configured to be located remote from the heart, such as in a pectoral region or other subcutaneous location. The electrode configuration defines a sensing vector between the electrodes is configured to perform far field sensing for cardiac activity of the heart, even though the device is located remote from the heart.

The cardiac activity signals are processed over one or more sensing channels by common or different configurations of sensing circuitry. For example, one sensing circuit may be configured to perform near field sensing, such as in connection with an electrode configuration located within or immediately adjacent chamber of the heart for which the cardiac activity is of interest. As another example, another sensing circuit may be configured to perform far field sensing, such as in connection with electrode configuration located outside of or remote from the chamber of the heart for which the cardiac activity is of interest.

According to the invention, at 504, the CA signals are filtered, by a filter within or coupled to the sensing circuitry, to separate a respiratory component from the CA signals. The respiratory component varies based on at least one of the respiration rate and/or respiration depth (e.g. tidal volume).

According to the invention, at 506, one or more processors analyze the respiration component for a respiration characteristic of interest (COI). For example, the respiration COI may be amplitude peaks and valleys in the respiration component. Additionally or alternatively, the respiration COI may relate to a slope of the respiration COI, such as when identifying maximum or minimum slopes, changes in slope, zero crossings and the like.

According to the invention, at 508, the one or more processors analyze the respiration COI to identify a respiration anomaly. Variation in the respiration COI is indicative of one or more respiration anomalies. For example, the variation in the respiration COI may correspond to at least one of variations in an amplitude of the respiratory component or variation in an interval within the respiratory component. For example, the interval within the signal component corresponds may represent a breathing cycle as indicated by a period between successive peaks or valleys of the signal component. Additionally or alternatively, the one or more processors may be further configured to determine the interval within the respiration component by counting a number of at least one of peaks or valleys in the respiratory component over a period of time. Additionally or alternatively, the one or more processors may be further configured to analyze the respiration component for at least one of an area under the curve, a slope, amplitude or intervals between peaks or valleys in connection with identifying the respiration COI.

The respiration components, respiration COI and respiration anomalies collectively represent respiration data that may more generally be utilized in accordance with embodiments herein in combination with other BGA data, IMD data and/or BRM data.

By way of example, the one or more processors is configured to analyze the respiratory component for a respiration characteristic of interest that identifies at least one of a respiration rate, a respiration depth, or respiration irregularity, that is indicative of at least one of hypopnea, sleep apnea, dyspnea, tachypnea, or bradypnea. Additionally or alternatively, the one or processors is configured to identify the respiration anomaly to be i) sleep apnea when the interval within the respiration component drops below an interval threshold, ii) hypopnea when the amplitude of the respiration component falls below an amplitude threshold, iii) dyspnea when the amplitude drops below an interval threshold and stays below the threshold for a longer period of time compared to sleep apnea, or iv) tachypnea based on a number of breaths greater than a normal range.

As a further example, the one or more processors may compare amplitudes of the peaks and/or valleys in the respiration component to one or more amplitude thresholds. The amplitude threshold may be preprogrammed by a clinician or automatically determined by the device, such as during a calibration operation or periodically throughout operation. The amplitude threshold is indicative of a minimum acceptable respiration depth, namely a level of inspiration and/or expiration (e.g. a minimum level for an acceptable shallow breath for which the patient would obtain sufficient oxygen). Optionally, one amplitude threshold may be utilized to distinguish hypopnea, while a second amplitude threshold may be utilized to distinguish apnea.

Additionally or alternatively, at 508, the one or more processors may compare an interval between successive peaks and/or successive valleys to one or more interval thresholds. The interval threshold may be preprogrammed by a clinician or automatically determined by the device during calibration or periodically. The interval threshold may be indicative of a minimum acceptable respiration rate, below which a patient is at risk of insufficient oxygen. Optionally, one interval threshold may be utilized to distinguish hypopnea, while a second interval threshold may be utilized to distinguish apnea.

The one or more processors may count a number of breaths, for which the amplitude falls below the amplitude threshold and/or for which the interval between successive breaths falls below the interval threshold. When a sufficient number of breaths satisfy one or both thresholds, the one or more processors may identify the condition to represent hypopnea or apnea. For example, when a long series of shallow breaths are identified at a relatively low respiration rate, processors may identify the rest ran anomaly to correspond to hypopnea. Additionally or alternatively, the one or more processors may determine when a predetermined period of time passes without detecting a breath having a respiration depth sufficient to exceed and apnea threshold. Additionally or alternatively, the one or more processors may determine when a relatively low number of breaths are detected during a predetermined period of time, thereby also potentially indicating sleep apnea.

At optional step 510, the one or more processors record the respiratory anomaly. Additionally or alternatively, the one or more processors may implement one or more actions based on the respiratory anomaly. Various actions are described elsewhere herein in connection with corresponding respiration anomalies. At optional step 512, in addition to or in place of the operation at 510, the one or more processors may be configured to provide an output in connection with the respiratory anomaly. Various outputs are described herein in connection with corresponding respiration anomalies.

Nonlimiting examples of actions or outputs include adjusting the parameters of an implantable medical device, initiating an operation to collect additional patient data, from the same device or from another device (e.g. another implantable medical device, a continuous glucose monitor, a FitBit^{™} device or other activity monitoring wearable device). Additionally or alternatively, the actions and/or outputs may include delivering and/or changing a therapy delivered by an external or implantable medical device, delivering or changing a drug regiment or dosage, automatically scheduling an appointment for the patient to meet their physician, schedule a particular follow-up diagnostic procedure (e.g. schedule a diagnostic imaging procedure), and the like. Nonlimiting examples of more acute actions or outputs include informing medical personnel that a patient is in immediate need of medical assistance, automatically dispatching an ambulance or other first responder to the patient, and the like. When the patient is admitted to or otherwise at resides at a medical or assisted-living facility, the respiration information alone or in combination with other medical information may be utilized to inform staff at the facility of a change in a patient's condition, including instances where the patient is in immediate need of medical attention (e.g. patient is experiencing apnea, a panic attack, hyperventilating, heart attack, has passed out, is experiencing a seizure etc.).

Figure 6 illustrates a process for collecting baseline information to be utilized subsequently for analyzing respiratory components for respiration anomalies in accordance with embodiments herein. The baseline information may include, among other things, thresholds, respiration patterns, breathing patterns, and the like.

At 602, the device collects CA signals to be used for calibration or to define a periodic baseline. At 604, baseline respiration components are separated from the baseline CA signals. At 606, the one or more processors analyze the baseline respiration components for one or more baseline COI. At 606, the one or more processors may also obtain information indicating a present physical condition of the patient (e.g. at rest, exercising), a patient posture, a degree to which the patient is attempting to breathe in a partial or full tidal volume and the like.

At 608, the one or more processors record the baseline COIs in connection with various patient condition information, such as posture, activity, the degree of tidal volume respiration and the like.

In connection with the calibration and baseline collection process of Figure 6, the patient may be instructed to stand, sit or lay down in a particular position and take a series of full breaths (e.g. with normal full inspiration and expiration as associated with defining the tidal volume) in a controlled and relaxed manner. The calibration or baseline respiration components may then be utilized to derive baseline COI, that are then used to set amplitude and/or interval thresholds (e.g. the baseline COI may be set as a multiple of the peak and valley amplitudes associated with a tidal volume). Additionally or alternatively, the patient may be instructed to take shallow breaths that are sufficient to avoid a feeling of being "out of breath". By collecting baseline respiration components in connection with a patient breathing in a shallow manner, the corresponding respiration COI may be used to directly set an amplitude and/or interval threshold.

Additionally or alternatively, calibration and/or baseline CA signals may be collected while a patient is undergoing a stress test or other physical activity. By collecting baseline respiration COI during a stress test or other physical activity, upper amplitude and/or interval thresholds may be defined. When the respiration COI exceeds the upper amplitude and/or interval thresholds, the condition may be interpreted as something other than physical activity, such as an anxiety attack, a heart attack or otherwise.

While the forgoing embodiments are described generally in connection with IMDs, it is understood that the methods and devices herein may be implemented in connection with portable non-lead-based wearable devices. For example, a portable non-lead-based wearable device maybe used with an infant in connection with avoiding sudden infant death syndrome (SIDS). A healthy infant will awake so as to resume breathing if the apnea lasts too long. If the infant is unable to awake itself, however, accidental suffocation and sudden death can occur. A portable non-lead-based wearable device may be used with any infant, child or adult who normally experience apnea at night. By way of example, the non-lead-based wearable device may be a device as described in U.S. Patent Publication 2015/0173670, "Method and Apparatus for Biometric Monitoring". It is recognized that the apparatus of the '670 application would need to be modified to collect CA signals and separate respiratory components as described herein.

### Integration of Respiratory Anomaly Monitoring with Digital Healthcare System

The foregoing embodiments are described generally in connection with an individual implantable medical device or portable non-lead based wearable device, however, embodiments herein are not so limited. Instead, the respiratory components, respiration COI and respiration anomalies may be monitored and provided to a larger digital healthcare system for integration with other types of medical information concerning a particular patient. The integration of the respiration information with other types of medical information may be utilized in a variety of manners, nonlimiting examples of which include adjusting the parameters of an implantable medical device, initiating additional data collection operations, delivering and/or changing a therapy delivered by a medical device, delivering or changing a drug regiment or dosage, scheduling an appointment for the patient to meet their physician, schedule a particular follow-up diagnostic procedure (e.g. schedule a diagnostic imaging procedure), and the like. Nonlimiting examples of more acute actions to be taken may include informing medical personnel that a patient is in immediate need of medical assistance, automatically dispatching an ambulance or other first responder to the patient, and the like. When the patient is admitted to or otherwise at resides at a medical or assisted-living facility, the respiration information alone or in combination with other medical information may be utilized to inform staff at the facility of a change in a patient's condition, including instances where the patient is in immediate need of medical attention (e.g. patient is experiencing apnea, a panic attack, hyperventilating, heart attack, has passed out, is experiencing a seizure etc.).

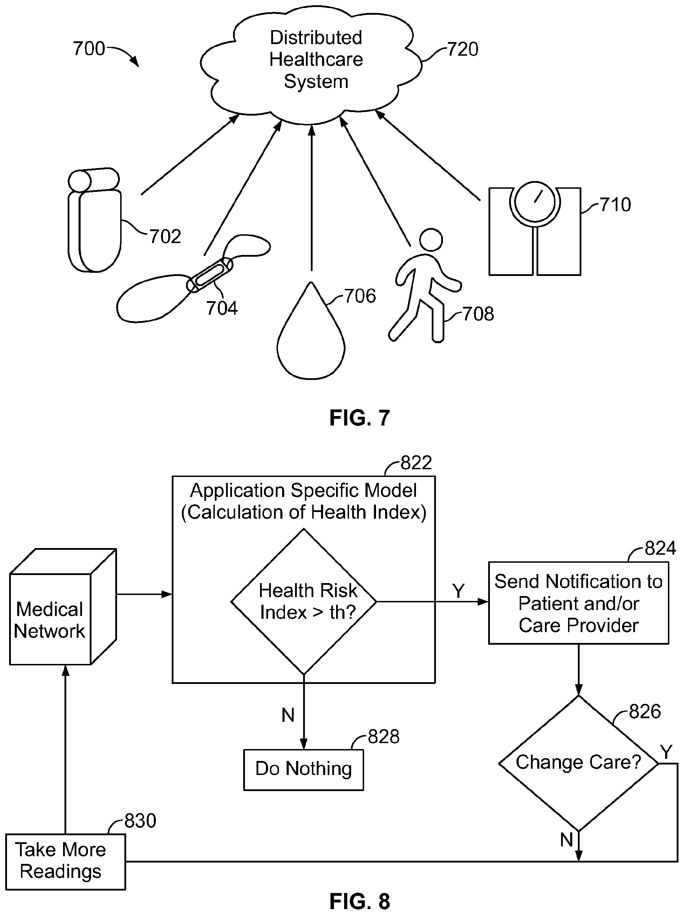

Figure 7 illustrates a block diagram of a system 700 for integrating external diagnostics with remote monitoring of data provided by implantable medical devices in accordance with embodiments herein. The system may be implemented with various architectures, that are collectively referred to as a healthcare system 720. By way of example, the healthcare system 720 may be implemented as described herein. The healthcare system 720 is configured to receive respiration components, respiration COI, respiration anomalies, as well as other medical data from a variety of external and implantable sources including, but not limited to, active IMDs 702 capable of delivering therapy to a patient, passive IMDs or sensors 704, BGA test devices 706, wearable sensors 708, and point-of-care (POC) devices 710 (e.g., at home or at a medical facility). The respiration information may be collected and analyzed by one or more of the devices illustrated in figure 7, including analyze the respiratory component to identify the respiration COI; and identify a respiration anomaly based on the respiration COI. When a respiration anomaly is identified, the identification may trigger or initiate various other test, actions and outputs by the various devices illustrated in Figure 7.

The data from one or more of the external and/or implantable sources is collected and transmitted to one or more secure databases within the healthcare system 720. Optionally, the patient and/or other users may utilize a patient data entry (PDE) device, such as a smart phone, tablet device, etc., to enter behavior related medical (BRM) data, such as during calibration or baseline determination for respiration parameters. A patient may also enter BRM data periodically, when experiencing certain breathing patterns, in connection with certain daily activities (e.g., exercise, mealtimes) and the like. For example, a patient may use a smart phone to provide feedback concerning activities performed by the patient, a patient diet, nutritional supplements and/or medications taken by the patient, how a patient is feeling (e.g., tired, dizzy, weak, good), etc. as one nonlimiting example, a patient's diet for a particular day and medication dosage may be correlated to a patient's breathing patterns while sleeping and/or more generally a patient's overall sleep pattern. For example, the system may identify that a patient's quality of sleep improves or detracts based on the time of day when a patient has their last meal, the calorie intake of the last meal or all meals for the day, whether the patient is ingesting excessive sugars too late in the day and the like. As another example, feedback could be provided at the time when a patient is beginning to have a midnight snack or late-night dessert (e.g. "you know if you eat that, you are not going to sleep well", "if you have ice cream now, you will be awake tonight from 2 AM to 4 AM"). Further nonlimiting examples of BRM data, as well as how to collect and utilize BRM data, are described in the U.S. Patent Application 2021/0020294.

Additionally or alternatively, when a breathing anomaly is detected (e.g. excessively fast breathing, excessively slow breathing, excessively shallow breaths), the breathing anomaly may automatically trigger an instruction for additional test to be performed or data to be collected. For example, the system may instruct a wearable or otherwise local external BGA test device 706 two automatically collect lab test results for specific tests and then transmit the lab test results to the healthcare system 720. The BGA test device 706 may be implemented at a variety of physical locations, such as one or more "core" laboratories, a physician's office, ER (emergency room), OR (operating room) and/or a medical facility POC (e.g., during hospitalizations or routine healthcare visits). The BGA test device 706 may be implemented as an at-home POC device 710 that collects test results periodically or continuously monitor one or more body generated analytes (e.g., blood glucose). The at home POC device 710 may transmit the raw BGA data to the medical network (e.g., a local external device and/or remote server). Additionally or alternatively, the at-home POC device 710 may implement a corresponding test of the BGA data for a characteristic of interest (COI) such as a malnutrition state COI, an electrolyte COI, a cardiac marker COI, a hematology COI, a blood gas COI, a coagulation COI, an endocrinology COI. The POC device 710 transmits the COI (and optionally the BGA data) to the healthcare system 720 as the tests are performed at home or elsewhere. The POC device 710 may implement periodic or continuous tests for glucose levels, such as through sensors and handheld devices offered under the trademark FREESTYLE LIBRE^{®} by Abbott Laboratories. Optionally, the BGA test device 706 may be implemented as a fully implantable "lab on a chip", such as an implantable biosensor array, that is configured to collect lab test results. The COI from the BGA data may be correlated with daytime and/or nighttime breathing patterns. Additionally or alternatively, the COI from the BGA data may be correlated with posture and/or activity data, as well as daytime or nighttime breathing patterns (e.g. to correlate a quality of sleep with the patient's levels of one or more COI from the BGA data).

Figure 8 illustrates a high-level flowchart of a method, implemented by a medical network, for processing respiration anomalies in connection with other medical devices that collect BGA data and/or IMD data in accordance with embodiments herein.

The healthcare system 720 includes one or more computing devices (e.g., servers, local external devices, MP devices) that are configured to collect and process IMD data (including respiration data) and/or BGA data. The example of Figure 8 represents an example of a high-level analysis that may be implemented, with more detailed examples provided herein. Upon receipt of new (or changes in) respiration data, BGA data and/or other IMD data, at 822, the processor of the computing device(s) identifies one or more application specific models or ASM to analyze the respiration data, BGA and/or IMD data. Optionally, the analysis by the ASM may incorporate the additional respiration data, BGA and/or IMD data into any relevant trend tracked in connection with the present patient. The application specific model may be implemented in various manners, as described herein, including but not limited to lookup tables, decision trees, machine learning algorithms and the like. At 822, the processor calculates a health risk index based on the incoming respiration data, BGA and/or IMD data, alone or in combination with previously stored respiration data, BGA and IMD data. The health risk index represents a general indicator of a degree to which the patient is experiencing a health state or potential health risk. As a patient's health deteriorates, indicated by one or more characteristics reflected in the BGA and IMD data, the health risk index will similarly elevate. As one nonlimiting example, the health risk index may represent a breathing anomaly indicator that tracks instances in which the patient experienced certain breathing anomalies (e.g. sleep apnea, hypopnea, shortness of breath, highly accelerated breathing). Additionally or alternatively, the health risk index may include an indication of a quality of sleep. The health risk index may be utilized to track trends and changes in the level or degree of the breathing anomaly and/or quality of sleep over time.

At 822, the processor may optionally generate a treatment diagnosis based on the respiration data, IMD data and BGA data. At 822, the processor also determines whether the health risk index exceeds one or more thresholds. When the health risk index does not exceed the threshold(s), the process interprets the condition as an indication that the incoming BGA, respiration, and/or other IMD data indicates that a patient's health condition remains relatively stable. This is assessed as either an instantaneous change relative to the last health risk index or based on a gradual increase in the health risk index over a pre-defined period of time. Accordingly, flow moves to 828 where the process determines that no other action is necessary. Alternatively, when the health risk index exceeds the threshold, the process interprets the condition as an indication that the incoming BGA, respiration and/or other IMD data indicates that a patient's health condition is deteriorating and in connection there with flow moves to 824.

At 824, the processor generates a treatment notification based on the treatment diagnosis and directs the treatment notification to be sent to the patient and/or a care provider. At 826, the one or more processors determine whether a change in care has been identified by the treatment diagnosis. Optionally, the operation at 826 may be implemented manually by a clinician or other medical practitioner. As a further option, the operation at 826 may be implemented automatically by one or more processors, as well as manually by a clinician or medical practitioner. The clinician or medical practitioner may then be afforded an option to "override" or modify the automated determination of a change in care.

At 830, the processor determines whether to obtain additional BGA, respiration and/or other IMD data and the process continues by collecting additional data. For example, the operation at 830 may simply represent a continuous loop at which the healthcare system waits to receive new/additional BGA, respiration and/or other IMD data. Additionally or alternatively, the processor may determine (e.g., as part of the treatment diagnosis) that further data should be obtained before a change in care is decided. Optionally, the operation at 830 may be implemented manually by a clinician or other medical practitioner. As a further option, the operation at 830 may be implemented automatically by one or more processors, as well as manually by a clinician or medical practitioner. The clinician or medical practitioner may then be afforded an option to "override" or modify the automated determination to obtain additional BGA, respiration and/or other IMD data. The process of Figure 8 automatically develops the treatment diagnosis (e.g., clinical insights) based on all available data. The clinical insights may result in a determination to i) collect more data, ii) recommend a change in clinical care or otherwise. Optionally, a clinician may be afforded the option to "Opt-In" or "Opt-out" of one or more different features and applications, thereby allowing the clinicians to choose which clinical insights they receive in connection with managing patients. Optionally, a clinician may be afforded the option to make decisions (e.g., render a diagnosis, change treatment, collect more data) and/or validate/reject decisions rendered automatically by the one or more processors.

Optionally, the process of Figure 8 may be implemented in whole or in part within one or more IMD, a PDE and/or a local external computing device. For example, an IMD may track the respiration data and/or other IMD data and detect possible deterioration of patient's health. When the IMD detects possible deterioration, the IMD notifies the patient to perform a POC measurement to collect supplemental BGA data. Optionally, when the IMD detects possible deterioration, the IMD may automatically convey a device command (as a treatment notification) to a BGA test device. In response, the BGA test device may automatically collect supplemental BGA data. The combination of the IMD data and the supplemental BGA data is analyzed in accordance with embodiments herein locally or remotely. Additionally or alternatively, the BGA test device may perform continuous BGA monitoring (e.g., instructions patient to take PAP measurement, etc.) and locally analyze the BGA data for indications of possible deterioration in a patient condition. When the BGA test device identifies a possible deterioration in a patient condition, the BGA test device may automatically convey a device command to an IMD to direct the IMD to collect supplemental IMD data. The combination of the BGA data and the supplemental IMD data is analyzed in accordance with embodiments herein locally or remotely.

### Healthcare System

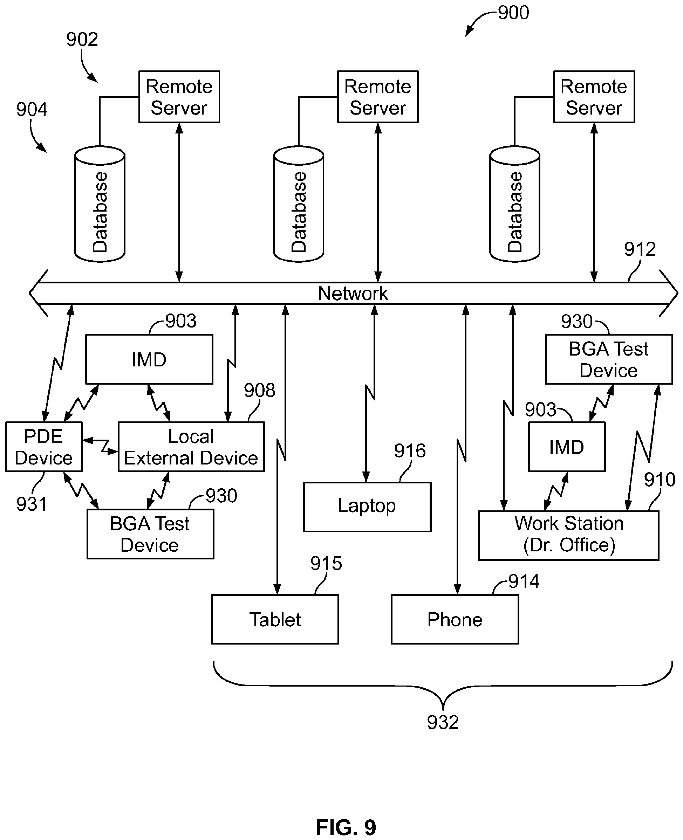

Figure 9 illustrates a healthcare system 900 formed in accordance with embodiments herein. The healthcare system 900 includes one or more servers 902, each of which is connected to one or more database 904. The servers 902 and databases 904 may be located in a cloud-based environment, at a common physical location and/or distributed between multiple remote locations within a city, state, country or worldwide. The system 900 also includes one or more IMDs 903, one or more local external devices 908, one or more BGA test devices 930, one or more PDE devices 931 and one or more medical personnel (MP) devices 932, all of which communicate (directly or indirectly) through the network 912 to the servers 902 and/or one another. The IMD 903 may be passive or active, may collect various types of data, such as cardiac electrical, respiration data and/or mechanical activity data, PAP or other pressure related data, impedance data, RPM data, flow data, and the like. The BGA test device 930 may analyze various types of body generated analytes to derive the BGA data. The PDE device 931 may communicate with any or all of the IMDs 903, local external devices 908, a BGA test device 930, as well as the network 912. The PDE device 931 collects BRM data, such as based on manual inputs from a patient or other user, and/or based on automatic video and/or audio monitoring.

The local external device 908 may be implemented as a variety of devices including, but not limited to, medical personnel programmer, a local RF transceiver and a user workstation, smart phone, tablet device, laptop computer, desktop computer and the like. The MP devices 932 may also be implemented as a variety of devices including, but not limited to, medical personnel programmer, workstation, smart phone, tablet device, laptop computer, desktop computer and the like.

The server 902 is a computer system that provides services to other computing systems over a computer network. The servers 902 control the communication of information including IMD data, patient entered data, medical record information and BGA. The servers 902 interface with the network 912 to transfer information between the servers 902, databases 904, local external devices 908, medical personnel devices 932 for storage, retrieval, data collection, data analysis, diagnosis, treatment recommendations and the like.

The databases 904 store all or various portions of the information described herein, including, but not limited to, respiration and/or other IMD data, BGA data, BRM data, medical record information, treatment diagnoses and recommendations, and the like. Various portions of the information may be downloaded or uploaded in combination or separately to/from the databases 904, local external devices 908 and MP devices 932. The local external device 908 may reside in a patient's home, a hospital, or a physician's office. The local external device 908 communicates wired or wirelessly with one or more IMD 903 and/or BGA test devices 930. The servers and devices described herein may wirelessly communicate with one another utilizing various protocols, such as Bluetooth, GSM, infrared wireless LANs, HIPERLAN, 3G, satellite, as well as circuit and packet data protocols, and the like. Alternatively, a hard-wired connection may be used to connect the servers and devices. The local external device 908, when implemented as a programmer, may be configured to acquire cardiac signals from the surface of a person (e.g., ECGs), and/or intra-cardiac electrogram (e.g., IEGM) signals from the IMD 903. The local external device 908 interfaces with the network 912 to upload the data and other information to the server 902.

Optionally, the local external device may represent a local RF transceiver that interfaces with the network 912 to upload IMD data and/or BGA data.

The workstation 910 may interface with the network 912 via the internet or POTS to download various data, information, diagnoses and treatment recommendations from the database 904. Alternatively, the workstation 910 may download raw data from the surface ECG units, leads, or monitoring device via either the programmer or the local RF transceiver. Once the user workstation 910 has downloaded the cardiac signal waveforms, ventricular and atrial heart rates, or detection thresholds, the user workstation 910 may process the information in accordance with one or more of the operations described above. The system may download information and notifications to the cell phone 914, the tablet device 915, the laptop 916, or to the server 902 to be stored on the database 904.

Thus, provided is a distributed "digital" healthcare system that collects various types of data, enables the data to be analyzed by various computing devices within the system and determines one or more treatment diagnosis and treatment recommendation substantially in real-time with the collection of new data. In this manner, unneeded and undesired hospitalizations may be avoided through preventative detection, reducing costs associated with emergency medical procedures. Additionally, such a system also assists in prolonging a human's life and increases patient care. Thus, an improved system and methodology are provided.

Optionally, the health risk index may be utilized to rank and schedule patients for future appointments to ensure those patients with the greatest risk of a medical emergency are monitored more closely than those with less of a risk.

Additionally or alternatively, a distributed healthcare system may be provided as described in the 62/875,870 provisional application. The system includes one or more PDE devices that communicate over a network with various other devices, such as IMDs, BGA test devices, MP devices, local external devices, servers and the like. Optionally, the PDE devices may communicate through a wholly or partially wired subsystem. The network may represent the World Wide Web, a local area network, a wide area network and the like. When the PDE device includes a GUI, the patient or other user may input patient data in addition to IMD data and BGA data. Optionally, the PDE devices may include one or more microphones that are configured to listen for audible information spoken by a user or patient, such as a verbal statement to enter patient data. Optionally, the PDE devices 960 may include one or more cameras that are configured to capture still images and/or video that is processed utilizing image recognition to identify what action a patient is performing (e.g., what, when and how much a patient is eating and/or drinking).

The user interface is configured to receive behavior related medical (BRM) data related to information indicative of an action or conduct by a patient that will affect one or more physiologic characteristics of interest and/or information indicative of a present state experienced by a patient in connection with a physiologic characteristic of interest. The user interface may include a variety of visual, audio, and/or mechanical devices. For example, the user interface can include a visual input device such as an optical sensor or camera, an audio input device such as a microphone, and a mechanical input device such as a keyboard, keypad, selection hard and/or soft buttons, switch, touchpad, touch screen, icons on a touch screen, a touch sensitive areas on a touch sensitive screen and/or any combination thereof. Similarly, the user interface can include a visual output device such as a liquid crystal display screen, one or more light emitting diode indicators, an audible output device such as a speaker, alarm and/or buzzer, and a mechanical output device such as a vibrating mechanism. The display may be touch sensitive to various types of touch and gestures. As further examples, the user interface may include a touch sensitive screen, a non-touch sensitive screen, a text-only display, a smart phone display, an audible output (e.g., a speaker or headphone jack), and/or any combination thereof. The user interface permits the user to select one or more of a switch, button or icon in connection with various operations of the PDE device in connection with entering the BRM data. As nonlimiting examples, the patient or a third-party (e.g., family member, caregiver) may enter, through the PDE device, information related to the patient's diet and/or nutritional supplements (e.g., what, when and how much a patient is taking), information concerning whether a patient is following a physician's instructions, information indicative of a present state experienced by the patient and the like. For example, a user may use a keyboard, touch screen and/or mouse to enter BRM data. Optionally, the user may enter the BRM data through spoken words (e.g., "Alexa I just took my medication", "Alexa I am eating 3 slices of peperoni pizza", "Alexa I am eating an apple", "Alexa I am drinking a 72 oz. soda and eating a candy bar).

Optionally, the PDE device may automatically monitor actions or conduct of interest. For example, a camera may be positioned to have a kitchen in a field of view. Still or video images from the camera are analyzed by one or more processors such as through image recognition to identify what, when and how much a patient eats or drinks. Optionally, the PDE device may include a microphone positioned near a kitchen and/or eating area. The audio recording may be analyzed by one or more processors to identify sounds indicative of eating and/or drinking food products of interest. The results from the analysis of the images and/or audio recording are saved as BRM data are utilized as explained herein. Optionally, the PDE device may automatically track actions by a patient, such as through the use of other types of sensors (e.g., refrigerator or kitchen cabinet door sensor, sensor on a treadmill). Optionally, the PDE device may include a position tracking device sold under the trademark FITBIT^{®} by Fitbit Inc. or other types of position tracking devices. The position tracking device may monitor and collect, as BRM data, movement information, such as a number of steps or distance traveled in a select period of time, a rate of speed, a level of exercise and the like. Optionally, the PDE device may monitor and collect, as BRM data, heart rate.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A medical device (100), comprising:
sensing circuitry (180) configured to obtain intracardiac electrogram, hereinafter referred to as IEGM, or electrogram, hereinafter referred to as ECG, signals indicative of cardiac activity over a series of beats and a number of breaths;
memory (152) configured to store program instructions; and
a processor (164), **characterized by:**
a filter (216) configured to separate, from the IEGM or ECG signals, a respiratory component that varies based on at least one of respiration rate or respiration depth over the number of breaths, wherein the processor (164) is configured, when executing the program instructions, to:
analyze the respiratory component to identify a respiration characteristic of interest (COI), the respiration COI based on at least one of variations, over the number of breaths, in an amplitude (415) of the respiratory component or an interval (416, 418) within the respiratory component; and
identify a respiration anomaly based on the respiration COI.

2. The medical device of claim 1, wherein the processor (164) is configured to analyze the respiratory component for the respiration COI identify at least one of a respiration rate, a respiration depth, or respiration irregularity, that is indicative of at least one of hypopnea, sleep apnea, dyspnea, tachypnea, bradypnea.

3. The medical device of claim 1 or 2, wherein the processor (164) is configured to identify the respiration anomaly to be i) sleep apnea when the interval within the respiration component drops below an interval threshold, or ii) hypopnea when the amplitude of the respiration component falls below an amplitude threshold.

4. The medical device of any one of the claims 1 to 3, wherein the filter (216) represents at least one of a band pass filter or a low-pass filter configured to separate the respiratory component from a cardiac activity component within the IEGM or ECG signals, wherein filter blocks signal components having a frequency of greater than 1 Hz.

5. The medical device of claim 4, wherein the filter (216) represents a band pass filter that removes ADC baseline component to avoid baseline wandering within the respiration component.

6. The medical device of any one of the claims 1 to 5, wherein the processor (216) is further configured to determine interval within the respiration component by counting a number of at least one of peaks (402, 403, 40, 405) or valleys (410, 411, 412, 413) in the respiratory component over a period of time.

7. The medical device of any one of the claims 1 to 6, wherein the processor (216) is configured to analyze the respiration component for at least one of an area under the curve, a slope, amplitude (415) or intervals (416, 418) between peaks (402, 403, 40, 405) or valleys (410, 411, 412, 413) in connection with identifying the respiration COI.

8. The medical device of any one of the claims 1 to 7, wherein the medical device (100) is an implantable medical device (100) and further comprises electrodes (114, 126) electrically connected to the sensing circuit (180), the electrodes (114, 126) are positioned with respect to a housing (102) of the implantable medical device (100), the electrodes (114, 126) defining a sensing vector along which the IEGM signals are sensed as far field electrogram signals.

9. The medical device of any one of the claims 1 to 8, wherein the interval within the signal component corresponds to a breathing cycle as indicated by a period between successive peaks or valleys of the signal component.

10. The medical device of any one of the claims 1 to 9, wherein the processor (164) is configured to at least one of perform an action or provide an output, including at least one of:
a) adjusting parameters of an implantable medical device (100),
b) initiating an operation to collect additional patient data, from the same device or from another device,
c) at least one of delivering or changing a therapy delivered by an external device (104) or the medical device (100),
d) delivering or changing a drug regiment or dosage,
e) automatically scheduling a patient-physician appointment,
f) scheduling a follow-up diagnostic procedure,
g) providing an output indicating that a patient is in immediate need of medical assistance,
h) providing an output request to automatically dispatching an ambulance or other first responder to the patient,
i) providing an output indicating a change in a patient's condition,
j) providing an output indicating a patient is experiencing at least one apnea, a panic attack, hyperventilating, heart attack, has passed out, or a seizure, or
k) tracking apnea burden over time.

11. A method, **characterized by:**
filtering intracardiac electrogram, hereinafter referred to as IEGM, or electrogram, hereinafter referred to as ECG, signals indicative of cardiac activity over a series of beats and a number of breaths to separate a respiratory component that varies based on at least one of respiration rate or respiration depth over the number of breaths;
analyzing the respiratory component to identify a respiration characteristic of interest (COI), the respiration COI based on at least one of variations, over the number of breaths, in an amplitude (415) of the respiratory component or an interval (416, 418) within the respiratory component; and
identifying a respiration anomaly based on the respiration COI.

12. The method of claim 11, further comprising analyzing the respiratory component for the respiration COI to identify at least one of a respiration rate, a respiration depth, or respiration irregularity, that is indicative of at least one of hypopnea, sleep apnea, dyspnea, tachypnea, or bradypnea.

13. The method of claim 11 or 12, further comprising identifying the respiration anomaly to be i) sleep apnea when the interval within the respiration component drops below an interval threshold, or ii) hypopnea when the amplitude of the respiration component falls below an amplitude threshold.

14. The method of any one of the claims 11 to 13, wherein the filtering includes:
a) applying at least one of a band pass filter or a low-pass filter to separate the respiratory component from a cardiac activity component within the IEGM or ECG signals, wherein filtering blocks signal components having a frequency of greater than 1 Hz; and/or.
applying a band pass filter that removes ADC baseline component to avoid baseline wandering within the respiration component.

15. The method of any one of the claims 11 to 14, further comprising:
a) comparing non-IEGM or ECG signals indicative of at least one of patient posture or patient activity to a threshold value and based on the comparing, initiating obtaining the IEGM or ECG signals; and/or
b) analyzing non-IEGM or ECG signals indicative of at least one of patient posture or patient activity for an activity COI, and identifying a sleep behavior pattern based on the activity COI and the respiration COI; and/or
c) combining non-IEGM or ECG signals indicative of at least one of patient posture or patient activity with the respiration COI over a period of time to define a trend in sleep quality.

## Patentansprüche

1. Medizinische Vorrichtung (100), umfassend:
Erfassungsschaltungsanordnung (180), die dazu konfiguriert ist, Signale eines intrakardialen Elektrogramms, nachstehend als IEGM bezeichnet, oder eines Elektrogramms, nachstehend als EKG bezeichnet, die eine Herzaktivität angeben, über eine Reihe von Schlägen und eine Anzahl von Atemzügen zu erlangen;
Speicher (152), der dazu konfiguriert ist, Programmanweisungen zu speichern; und
einen Prozessor (164), **gekennzeichnet durch:**
einen Filter (216), der dazu konfiguriert ist, aus den IEGM- oder EKG-Signalen eine Atmungskomponente abzutrennen, die basierend auf mindestens einem von einer Atmungsrate oder einer Atmungstiefe über die Anzahl von Atemzügen variiert, wobei der Prozessor (164) beim Ausführen der Programmanweisungen zu Folgendem konfiguriert ist:
Analysieren der Atmungskomponente, um ein Atmungsmerkmal von Interesse (Atmungs-COI) zu identifizieren, wobei das Atmungs-COI auf mindestens einem von Variationen, über die Anzahl von Atemzügen, in einer Amplitude (415) der Atmungskomponente oder einem Intervall (416, 418) innerhalb der Atmungskomponente basiert; und
Identifizieren einer Atmungsanomalie basierend auf dem Atmungs-COI.

2. Medizinische Vorrichtung nach Anspruch 1, wobei der Prozessor (164) dazu konfiguriert ist, die Atmungskomponente auf das Atmungs-COI zu analysieren, um mindestens eines von einer Atmungsrate, einer Atmungstiefe oder einer Atmungsunregelmäßigkeit zu identifizieren, die mindestens eines von Hypopnoe, Schlafapnoe, Dyspnoe, Tachypnoe, Bradypnoe angibt.

3. Medizinische Vorrichtung nach Anspruch 1 oder 2, wobei der Prozessor (164) dazu konfiguriert ist, die Atmungsanomalie als i) Schlafapnoe, wenn das Intervall innerhalb der Atmungskomponente unter eine Intervallschwelle fällt, oder ii) Hypopnoe, wenn die Amplitude der Atmungskomponente unter eine Amplitudenschwelle sinkt, zu identifizieren.

4. Medizinische Vorrichtung nach einem der Ansprüche 1 bis 3, wobei der Filter (216) mindestens einen von einem Bandpassfilter oder einem Tiefpassfilter darstellt, der dazu konfiguriert ist, die Atmungskomponente aus einer Herzaktivitätskomponente innerhalb der IEGM- oder EKG-Signale abzutrennen, wobei der Filter Signalkomponenten blockiert, die eine Frequenz von über 1 Hz aufweisen.

5. Medizinische Vorrichtung nach Anspruch 4, wobei der Filter (216) einen Bandpassfilter darstellt, der eine ADC-Grundlinienkomponente entfernt, um ein Grundlinienwandern innerhalb der Atmungskomponente zu vermeiden.

6. Medizinische Vorrichtung nach einem der Ansprüche 1 bis 5, wobei der Prozessor (216) ferner dazu konfiguriert ist, ein Intervall innerhalb der Atmungskomponente durch Zählen einer Anzahl von mindestens einem von Wellenbergen (402, 403, 40, 405) oder Wellentälern (410, 411, 412, 413) in der Atmungskomponente über einen Zeitraum zu bestimmen.

7. Medizinische Vorrichtung nach einem der Ansprüche 1 bis 6, wobei der Prozessor (216) dazu konfiguriert ist, die Atmungskomponente auf mindestens eines von einer Fläche unterhalb der Kurve, einer Steigung, Amplitude (415) oder Intervallen (416, 418) zwischen Wellenbergen (402, 403, 40, 405) oder Wellentälern (410, 411, 412, 413) in Verbindung mit dem Identifizieren des Atmungs-COI zu analysieren.

8. Medizinische Vorrichtung nach einem der Ansprüche 1 bis 7, wobei die medizinische Vorrichtung (100) eine implantierbare medizinische Vorrichtung (100) ist und ferner Elektroden (114, 126) umfasst, die elektrisch mit der Erfassungsschaltung (180) verbunden sind, wobei die Elektroden (114, 126) in Bezug auf ein Gehäuse (102) der implantierbaren medizinischen Vorrichtung (100) positioniert sind, wobei die Elektroden (114, 126) einen Erfassungsvektor definieren, entlang dessen die IEGM-Signale als Fernfeldelektrogrammsignale erfasst werden.

9. Medizinische Vorrichtung nach einem der Ansprüche 1 bis 8, wobei das Intervall innerhalb der Signalkomponente einem Atemzyklus, wie durch eine Dauer zwischen aufeinanderfolgenden Wellenbergen oder Wellentälern der Signalkomponente angegeben, entspricht.

10. Medizinische Vorrichtung nach einem der Ansprüche 1 bis 9, wobei der Prozessor (164) dazu konfiguriert ist, mindestens entweder eine Handlung durchzuführen oder eine Ausgabe bereitzustellen, was mindestens eines von Folgenden enthält:
a) Einstellen von Parametern einer implantierbaren medizinischen Vorrichtung (100),
b) Einleiten eines Vorgangs zum Sammeln zusätzlicher Patientendaten aus der gleichen Vorrichtung oder aus einer anderen Vorrichtung,
c) mindestens eines von Abgeben oder Ändern einer Therapie, die durch eine externe Vorrichtung (104) oder die medizinische Vorrichtung (100) abgegeben wird,
d) Abgeben oder Ändern eines Medikamentenschemas oder einer Medikamentendosierung,
e) automatisches Planen eines Patienten-Arzt-Termins,
f) Planen eines weiteren diagnostischen Vorgangs,
g) Bereitstellen einer Ausgabe, die angibt, dass ein Patient sofort medizinische Hilfe benötigt,
h) Bereitstellen einer Ausgabeanforderung, um automatisch einen Krankenwagen oder einen anderen Rettungsdienst zu dem Patienten zu senden,
i) Bereitstellen einer Ausgabe, die eine Änderung eines Zustands des Patienten angibt,
j) Bereitstellen einer Ausgabe, die angibt, dass ein Patient gerade mindestens eine Apnoe, eine Panikattacke, Hyperventilieren, einen Herzanfall, eine Ohnmacht oder einen Krampfanfall erleidet oder
k) Verfolgen der Apnoelast im Zeitverlauf.

11. Verfahren, **gekennzeichnet durch:**
Filtern von Signalen eines intrakardialen Elektrogramms, nachstehend als IEGM bezeichnet, oder eines Elektrogramms, nachstehend als EKG bezeichnet, die eine Herzaktivität angeben, über eine Reihe von Schlägen und eine Anzahl von Atemzügen, um eine Atmungskomponente abzutrennen, die basierend auf mindestens einem von einer Atmungsrate oder einer Atmungstiefe über die Anzahl von Atemzügen variiert;
Analysieren der Atmungskomponente, um ein Atmungsmerkmal von Interesse (Atmungs-COI) zu identifizieren, wobei das Atmungs-COI auf mindestens einem von Variationen, über die Anzahl von Atemzügen, in einer Amplitude (415) der Atmungskomponente oder einem Intervall (416, 418) innerhalb der Atmungskomponente basiert; und
Identifizieren einer Atmungsanomalie basierend auf dem Atmungs-COI.

12. Verfahren nach Anspruch 11, ferner umfassend Analysieren der Atmungskomponente auf das Atmungs-COI, um mindestens eines von einer Atmungsrate, einer Atmungstiefe oder einer Atmungsunregelmäßigkeit zu identifizieren, die mindestens eines von Hypopnoe, Schlafapnoe, Dyspnoe, Tachypnoe, Bradypnoe angibt.

13. Verfahren nach Anspruch 11 oder 12, ferner umfassend Identifizieren der Atmungsanomalie als i) Schlafapnoe, wenn das Intervall innerhalb der Atmungskomponente unter eine Intervallschwelle fällt, oder ii) Hypopnoe, wenn die Amplitude der Atmungskomponente unter eine Amplitudenschwelle sinkt.

14. Verfahren nach einem der Ansprüche 11 bis 13, wobei das Filtern Folgendes enthält:
a) Anwenden mindestens eines von einem Bandpassfilter oder einem Tiefpassfilter, um die Atmungskomponente aus einer Herzaktivitätskomponente innerhalb der IEGM- oder EKG-Signale abzutrennen, wobei das Filtern Signalkomponenten blockiert, die eine Frequenz von über 1 Hz aufweisen; und/oder.
Anwenden eines Bandpassfilters, der eine ADC-Grundlinienkomponente entfernt, um ein Grundlinienwandern innerhalb der Atmungskomponente zu vermeiden.

15. Verfahren nach einem der Ansprüche 11 bis 14, ferner umfassend:
a) Vergleichen von Nicht-IEGM- oder -EKG-Signalen, die mindestens eines von einer Patientenhaltung oder Patientenaktivität angeben, mit einem Schwellenwert und auf der Grundlage des Vergleichens Einleiten eines Erlangens der IEGM- oder EKG-Signale; und/oder
b) Analysieren von Nicht-IEGM- oder -EKG-Signalen, die mindestens eines von einer Patientenhaltung oder Patientenaktivität angeben, auf ein Aktivitäts-COI und Identifizieren eines Schlafverhaltensmusters auf der Grundlage des Aktivitäts-COI und des Atmungs-COI; und/oder
c) Kombinieren von Nicht-IEGM- oder -EKG-Signalen, die mindestens eines von einer Patientenhaltung oder Patientenaktivität angeben, mit dem Atmungs-COI über einen Zeitraum, um einen Trend in der Schlafqualität zu definieren.

## Revendications

1. Dispositif médical (100), comprenant :
un circuit de détection (180) configuré pour obtenir des signaux d'électrogramme intracardiaque, ci-après appelé IEGM, ou d'électrogramme, ci-après appelé ECG, indicatifs de l'activité cardiaque sur une série de battements et un certain nombre de respirations ;
une mémoire (152) configurée pour stocker des instructions de programme ; et
un processeur (164), **caractérisé par** :
un filtre (216) configuré pour séparer, des signaux IEGM ou ECG, une composante respiratoire qui varie sur la base d'au moins l'un parmi le rythme de respiration ou la profondeur de respiration sur le nombre de respirations, dans lequel le processeur (164) est configuré, lors de l'exécution des instructions de programme, pour :
analyser la composante respiratoire pour identifier une caractéristique d'intérêt (COI) de respiration, la COI de respiration étant basée sur au moins l'une des variations, sur le nombre de respirations, d'une amplitude (415) de la composante respiratoire ou d'un intervalle (416, 418) dans la composante respiratoire ; et
identifier une anomalie de respiration sur la base de la COI de respiration.

2. Dispositif médical selon la revendication 1, dans lequel le processeur (164) est configuré pour analyser la composante respiratoire pour la COI de respiration pour identifier au moins l'un parmi un rythme de respiration, une profondeur de respiration ou une irrégularité de respiration, qui est indicatif d'au moins l'une parmi une hypopnée, une apnée du sommeil, une dyspnée, une tachypnée, une bradypnée.

3. Dispositif médical selon la revendication 1 ou 2, dans lequel le processeur (164) est configuré pour identifier l'anomalie de respiration comme étant i) une apnée du sommeil lorsque l'intervalle dans la composante de respiration descend en dessous d'un seuil d'intervalle, ou ii) une hypopnée lorsque l'amplitude de la composante de respiration tombe en dessous d'un seuil d'amplitude.

4. Dispositif médical selon l'une quelconque des revendications 1 à 3, dans lequel le filtre (216) représente au moins l'un parmi un filtre passe-bande ou un filtre passe-bas configuré pour séparer la composante respiratoire d'une composante d'activité cardiaque dans les signaux d'IEGM ou d'ECG, dans lequel le filtre bloque des composantes de signal ayant une fréquence supérieure à 1 Hz.

5. Dispositif médical selon la revendication 4, dans lequel le filtre (216) représente un filtre passe-bande qui supprime une composante de ligne de base d'ADC pour éviter une déviation de ligne de base dans la composante de respiration.

6. Dispositif médical selon l'une quelconque des revendications 1 à 5, dans lequel le processeur (216) est en outre configuré pour déterminer l'intervalle dans la composante de respiration en comptant un nombre d'au moins un parmi des pics (402, 403, 40, 405) ou des creux (410, 411, 412, 413) dans la composante respiratoire pendant une période donnée.

7. Dispositif médical selon l'une quelconque des revendications 1 à 6, dans lequel le processeur (216) est configuré pour analyser la composante de respiration pour au moins l'un parmi une surface sous la courbe, une pente, une amplitude (415) ou des intervalles (416, 418) entre des pics (402, 403, 40, 405) ou des creux (410, 411, 412, 413) en relation avec l'identification de la COI de respiration.

8. Dispositif médical selon l'une quelconque des revendications 1 à 7, dans lequel le dispositif médical (100) est un dispositif médical implantable (100) et comprend en outre des électrodes (114, 126) connectées électriquement au circuit de détection (180), les électrodes (114, 126) sont positionnées par rapport à un boîtier (102) du dispositif médical implantable (100), les électrodes (114, 126) définissant un vecteur de détection le long duquel les signaux IEGM sont détectés sous forme de signaux d'électrogramme de champ lointain.

9. Dispositif médical selon l'une quelconque des revendications 1 à 8, dans lequel l'intervalle dans la composante de signal correspond à un cycle respiratoire comme indiqué par une période entre des pics ou des creux successifs de la composante de signal.

10. Dispositif médical selon l'une quelconque des revendications 1 à 9, dans lequel le processeur (164) est configuré pour exécuter au moins une action ou fournir une sortie, comportant au moins l'un des éléments suivants :
a) le réglage de paramètres d'un dispositif médical implantable (100),
b) le lancement d'une opération de collecte de données de patient supplémentaires, à partir du même dispositif ou à partir d'un autre dispositif,
c) au moins l'une parmi l'administration ou la modification d'une thérapie administrée par un dispositif externe (104) ou le dispositif médical (100),
d) l'administration ou la modification d'une posologie ou d'un régime médicamenteux,
e) la planification automatique d'un rendez-vous patient-médecin,
f) la planification d'une procédure de diagnostic de suivi,
g) la fourniture d'un résultat indiquant qu'un patient a un besoin immédiat d'une assistance médicale,
h) la fourniture d'une demande de sortie pour envoyer automatiquement une ambulance ou un autre premier intervenant au patient,
i) la fourniture d'une sortie indiquant un changement dans l'état d'un patient,
j) la fourniture d'une sortie indiquant qu'un patient souffre d'au moins une apnée, une crise de panique, une hyperventilation, une crise cardiaque, un évanouissement ou une convulsion, ou
k) le suivi de la charge de l'apnée au fil du temps.

11. Procédé, **caractérisée par** :
le filtrage de signaux d'électrogramme intracardiaque, ci-après appelé IEGM, ou d'électrogramme, ci-après appelé ECG, indicatifs de l'activité cardiaque sur une série de battements et un certain nombre de respirations pour séparer une composante respiratoire qui varie sur la base d'au moins l'un parmi un rythme de respiration ou une profondeur de respiration sur le nombre de respirations ;
l'analyse de la composante respiratoire pour identifier une caractéristique d'intérêt (COI) de respiration, la COI de respiration étant basée sur au moins l'une des variations, sur le nombre de respirations, d'une amplitude (415) de la composante respiratoire ou d'un intervalle (416, 418) dans la composante respiratoire ; et
l'identification d'une anomalie de respiration sur la base de la COI de respiration.

12. Procédé selon la revendication 11, comprenant en outre l'analyse de la composante respiratoire pour la COI de respiration pour identifier au moins l'un parmi un rythme de respiration, une profondeur de respiration ou une irrégularité de respiration, qui est indicatif d'au moins l'une parmi une hypopnée, une apnée du sommeil, une dyspnée, une tachypnée, ou bradypnée.

13. Procédé selon la revendication 11 ou 12, comprenant en outre l'identification de l'anomalie de respiration comme étant i) une apnée du sommeil lorsque l'intervalle dans la composante de respiration descend en dessous d'un seuil d'intervalle, ou ii) une hypopnée lorsque l'amplitude de la composante de respiration tombe en dessous d'un seuil d'amplitude.

14. Procédé selon l'une quelconque des revendications 11 à 13, dans lequel le filtrage comporte :
a) l'application d'au moins l'un parmi un filtre passe-bande ou un filtre passe-bas pour séparer la composante respiratoire d'une composante d'activité cardiaque dans les signaux d'IEGM ou d'ECG, dans lequel le filtrage bloque des composantes de signal ayant une fréquence supérieure à 1 Hz ; et/ou
l'application d'un filtre passe-bande qui supprime la composante de ligne de base d'ADC pour éviter une déviation de ligne de base dans la composante de respiration.

15. Procédé selon l'une quelconque des revendications 11 à 14, comprenant en outre :
a) la comparaison de signaux non IEGM ou ECG indicatifs d'au moins l'une parmi une posture du patient ou l'activité du patient à une valeur seuil et, sur la base de la comparaison, le lancement de l'obtention des signaux d'IEGM ou d'ECG ; et/ou
b) l'analyse de signaux non IEGM ou ECG indicatifs d'au moins l'une parmi une posture du patient ou une activité du patient pour une COI d'activité, et l'identification un modèle de comportement de sommeil sur la base de la COI d'activité et de la COI de respiration ; et/ou
c) la combinaison de signaux non IEGM ou ECG indicatifs d'au moins l'une parmi une posture du patient ou une activité du patient avec la COI de respiration pendant une période de temps pour définir une tendance dans la qualité du sommeil.
